# EUROPEAN PATENT APPLICATION

(11) **EP 1 736 780 A1**
(43) Date of publication of application: **27.12.2006**
(21) Application number: 05447148.7
(22) Date of filing: 24.06.2005
(51) Int. Cl.: G01N 33/68, C12Q 1/48, G01N 33/543, C12Q 1/68

(54) **Method and means for detecting and/or quantifying hierarchical molecular change of a cell in response to an external stimulus**

(71) Applicant: Eppendorf Array Technologies S.A., 5000 Namur (BE)
(72) Inventor: Remacle, José, 5020 Malonne (BE); Bertholet, Vincent, 5100 Jambes (BE); Margaine, Sylvain, 5000 Namur (BE); Delongueville, Françoise, 5360 Natoye (BE); Van Huffel, Christophe, 1170 Watermael-Boitsfort (BE); Mainfroid, Véronique, 4300 Waremme (BE); Plennevaux, Christelle, 5640 St. Gérard (BE)
(74) Representative: Van Malderen, Joëlle

(57) **Abstract**

The present invention is related to a method for detecting and/or quantifying hierarchical (regulated) molecular changes of a cell in response to any external stimulus.

## Description

### Field of the invention

The present invention is related to a method for detecting and/or quantifying hierarchical (regulated) molecular change of a cell in response to an external biological, physical and/or chemical stimulus.

The present invention is also related to the kit (including kit of parts) comprising the various media and means for performing the steps of said method.

The present invention is also related to the computer system for performing specific steps of the method according to the invention.

A last aspect of the present invention is related to an apparatus, possibly an automate, for performing the various steps of the method according to the invention and possibly integrating the computer system according to the invention.

### Background of the invention

The study of complex living systems like the reactions and adaptation occurring within cells is difficult because of the high complexity of the cell, the involvement of large number of cell components, the various levels of regulation with interconnected relationships between these components and the very low amount of material usually available for the analysis. The assays for multiple components are now performed on a miniaturization scale by genomic studies for the expressed genes and by proteomic for proteins.

Indeed, any stimulus applied upon the cell requires a fast and adapted response by target cells which is mediated through different regulatory levels before being translated into mRNA and protein synthesis. One of the main common cell response is achieved through the stimulation of transduction cascades mostly through receptors activation, which relay at the intracellular level the extra cellular signals sensed by the cells. On the other side fast activation is based on modifications of pre-existing proteins. Transduction cascades culminate in the activation of transcription factors which constitute a particularly important class of proteins, as they make the junction between proteomics and genomics due on one side to their direct activation by signaling cascades and on the other side to the regulation of genes which are under their control. Gene expression is a later event, which is assessed by different methods being part of the genomic in its broad sense. Genes are then translated into proteins which are then processed by a series of post-translational modifications; the study of proteins and their modification being part of the proteomic analysis.

Understanding the whole process of cell biology requires the concerted analysis of all regulation levels, and their spatiotemporal integration, as they are linked to each other by a complex network of interactions with some positive or negative feedback occurring at different levels involving different cell compartments and having different time frames after a given stimulus or external input. This multilevel analysis is necessary to understand the cell response, as a single transduction cascade can be activated by a multitude of stimuli or stresses, and since a same stress can lead to the parallel activation of many cascades that are either interconnected, parallel each other and even compensate for one another. Similarly, the same transcription factors can differentially regulate several genes, with an overall effect positive, negative, or even hardly detectable, depending on the gene and the presence of other transcriptional factors.

### State of the art

Understanding biology as an overall well structured and regulated system requires examining the structure and dynamics of cellular function, rather than the characteristics of isolated parts of a cell. Identification of gene-regulatory networks is a major challenge.

Genomic and proteomic approaches for a global analysis of cell signalling have been described (Zhu and Snyder 2002, Current Opinion in Cell Biology, 14, 173-179). The US 2004/0096815 and US 2004/0096816 also compared proteomic and transcriptomic analysis for identifying a biological parameter which is modified following a stress or further to study cell ageing.

One of the most powerful methods for analysing a specific cellular response to a situation such as a stimulus is the use of DNA microarray technology to analyse multiple gene expression (Schena et al. 1995, Science, 270, 467-470). The ability to analyse many transcripts simultaneously provides a detailed molecular phenotype, which can be used to deduce the different subsets of responses affected upon activation or inactivation of a signalling pathways.

Although DNA microarray technologies have proven to be very powerful in analysing protein function and pathways, gene function is manifested by the activity of its protein product. Therefore, analysis of proteins is expected to be more informative for dissecting protein function and pathways. This is particularly true for signalling pathways, because many pathways mediate their effects through post-translational modification such as protein phosphorylation. Innovative approaches, including yeast two-hybrid, two-dimensional gel/mass spectrometry and protein microarray methods have emerged recently for the large-scale analysis of protein signalling pathways. Protein microarrays are generally antibody arrays in which antibodies directed against different proteins or epitopes are spotted onto a slide. Beside protein arrays, the most widely used proteomic analytical method is the separation of the proteins in 2D gel and the identification of the individual proteins based on their molecular weight or their particular sequences using mass spectroscopy.

Despite the obvious attraction of parallel profiling of transcripts and proteins on a global 'omic' scale, there are differences involved in their performance and their biological signification. "Transcriptomics" is now a robust technology capable of simultaneously quantifying hundreds or thousands of defined mRNA species in a miniaturized format. Conversely, proteomic analysis is currently much more limited in breadth and depth of coverage owing to variation in protein abundance, hydrophobicity, stability, size and charge.

Modeling the transcriptomic or proteomic multiparametric data has been proposed on one level of analysis mainly the interconnection of expressed genes or proteins according to their function and involvement in a biological situation. This new modeling is referred as system biology (Barabasi and Oltvai, 2004, Nature Reviews Genetics, 5, 21-33. It also includes some knowledge of the possible regulatory pathways but not performed on the same cell. So there is a need for method and means for performing multiparametric molecular assays on a cell at different levels of regulation and to integrate the data of a cell into a hierarchical view of a cell regulation.

### Summary of the invention

The aim of the present invention is to provide a solution to the comprehension of a cell in a particular situation as being a highly complex regulated system.

Until now, it was not possible to obtain a precise view of all possible interactions of the relationships (at defined time periods) between the different cell levels of regulation and the consequences of such regulation on the transcriptome and proteome of the cell submitted to a particular stimulus.

The invention proposed a solution to this problem and is related to a method and means for the detection and/or quantification of hierarchical (regulated) molecular change of a cell in response to any external stimulus by integrating at least two levels of cell function, the molecules involved in the first level being regulatory molecules interacting with the expression of the molecules of the second level, by the following steps:
a) obtaining a cell extract from the cell;
b) determining and/or quantifying the molecular change at a first level of the cell function by submitting the cell extract to a first assay upon microarray, said first assay capable of obtaining (comprising) the steps of:
   - a detection and/or a quantification of at least 5 different (activated) transcriptional factors (TF) present in the cell extract and/or,
   - a detection and/or a quantification of at least 5 different activated MAP-kinases (MAPK), including quantification of MAP-kinase activity;
c) detecting and/or quantifying the molecular cell changes at a second level of a cell function, by submitting the same cell extract to a second assay upon microarray, said second assay comprising :
   - a detection and/or a quantification of at least 10 different expressed genes and/or,
   - a detection and/or a quantification of at least 10 different corresponding proteins encoded by these expressed genes, wherein the expressed genes and the corresponding synthesized proteins are regulated by the activated transcriptional factors and MAP-kinases of the first level of the cell function;
d) collecting the results of the detection and/or the quantification of the expressed genes and/or synthesized proteins and the results of the detection and/or the quantification of the (activated) transcriptional factors and/or activated MAP-kinases;
e) correlating the (collected) results of the detection and/or the quantification of the expressed genes and/or synthesized proteins to the (collected) results of the detection and/or the quantification of the (activated) transcriptional factors and/or activated MAP-kinases;
f) integrating the (collected) results of said detection and/or quantification and their correlation in a model of (regulated) hierarchical molecular change, preferably by a computer and possibly,
g) characterizing (preferably by a computer) the hierarchical (regulated) molecular changes in the cell resulting from the external biological, physical and/or chemical stimulus.

The present invention is also related to means and media for obtaining this detection, such as a kit or kit of parts or device comprising means and media for performing the detection and/or quantification method steps.

The present invention also concerns the kit or device comprising a computer program collecting and integrating the results of this detection and/or quantification and their correlation, and possibly for characterizing hierarchical (regulated) molecular change in a cell (preferably resulting from the stimulus). This computer program (product) could also be used for characterizing the relationships between the different levels of regulation occurring in the cell, and for providing to the experimenter, information regarding these various interactions.

The present invention will be described in more details in the following examples, in reference to the enclosed figures presented as non-limiting illustrations of the various embodiments of the present invention.

### Short description of the figures and tables

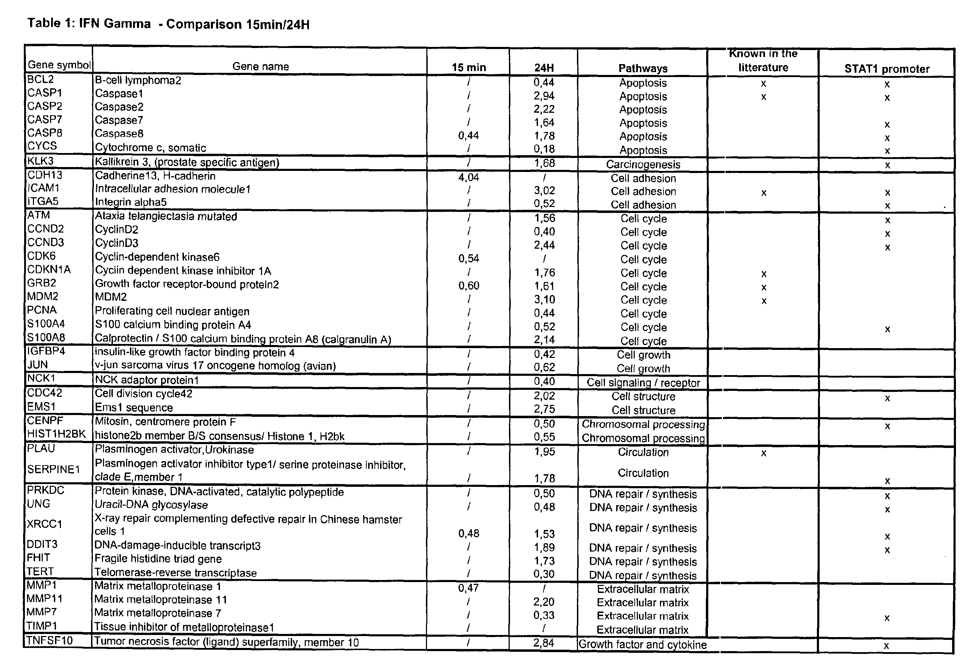

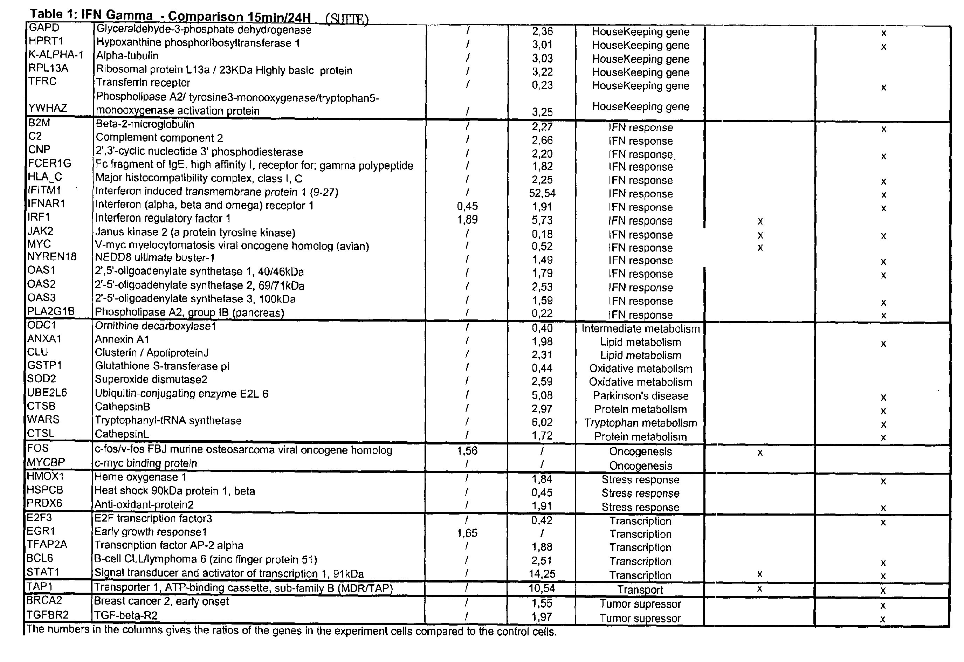

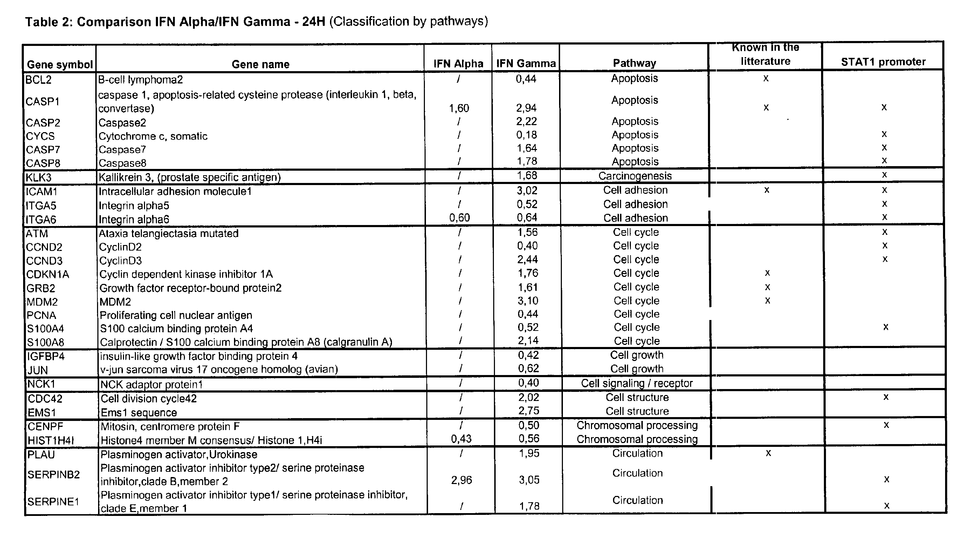

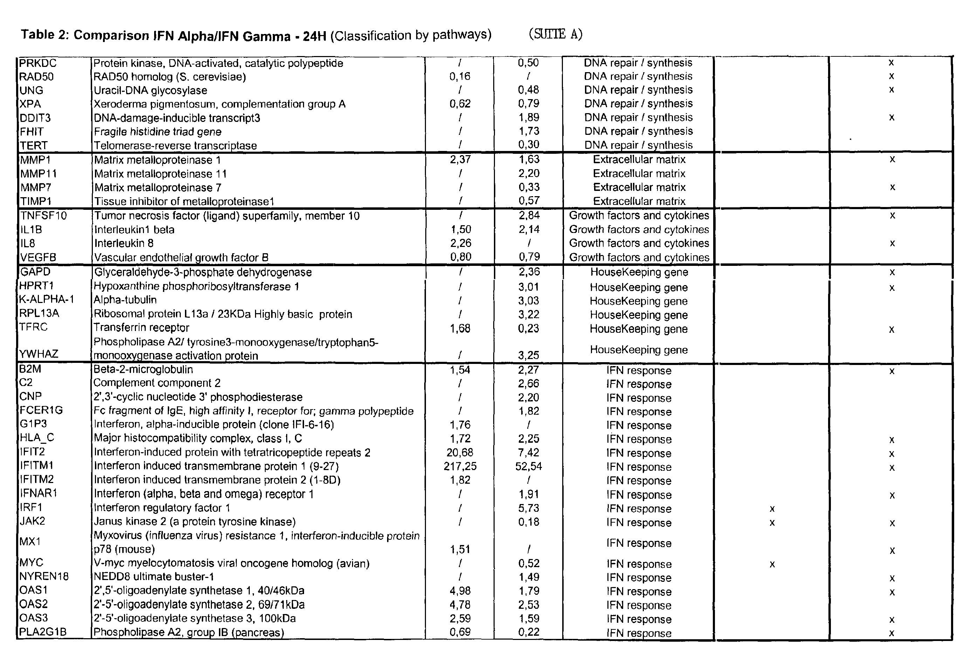

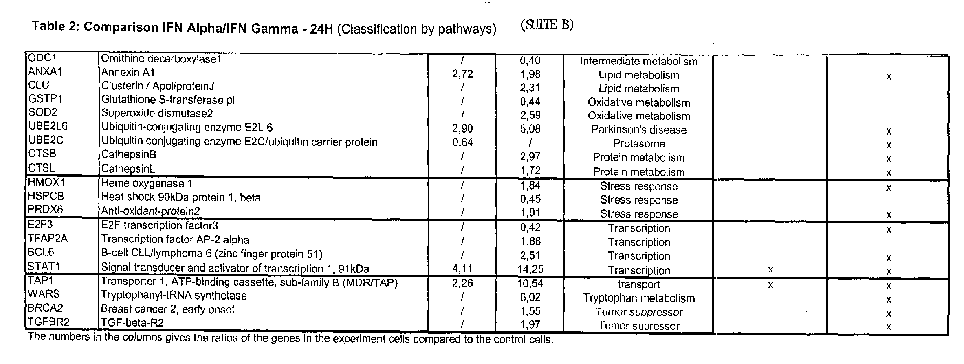

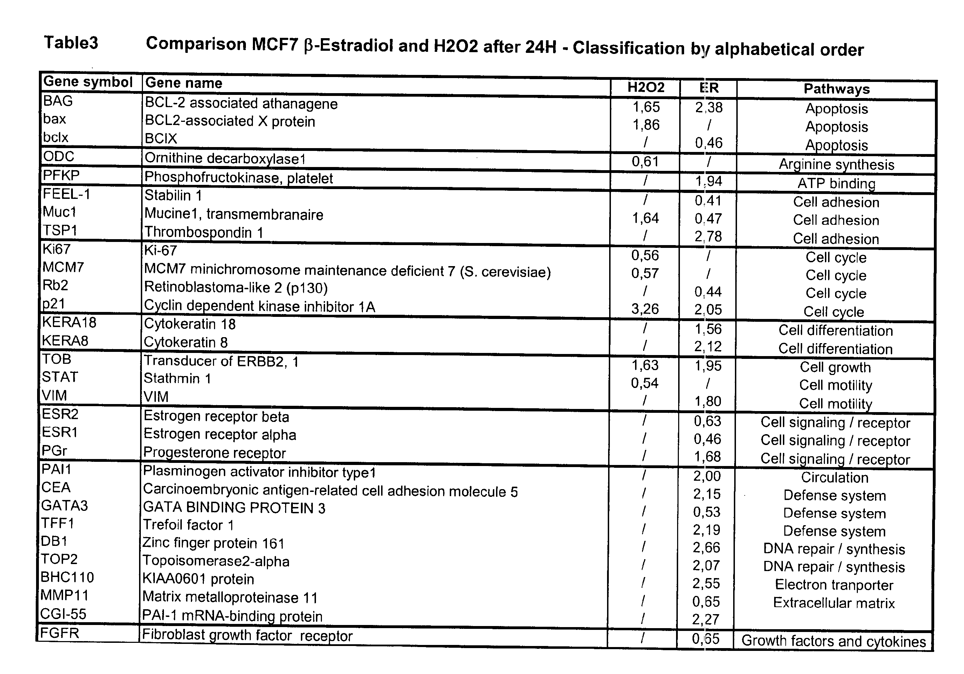

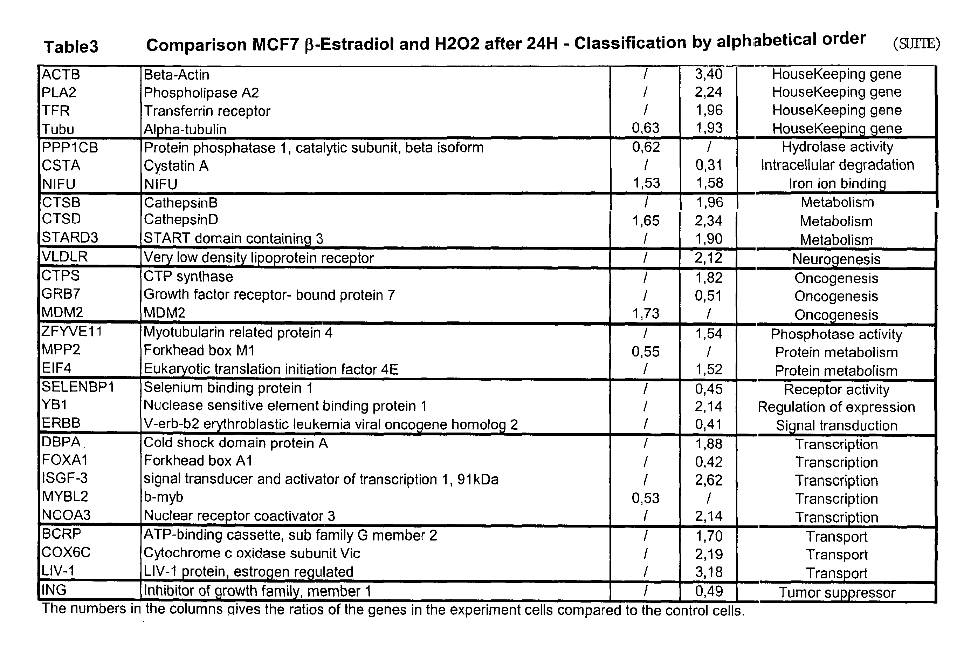

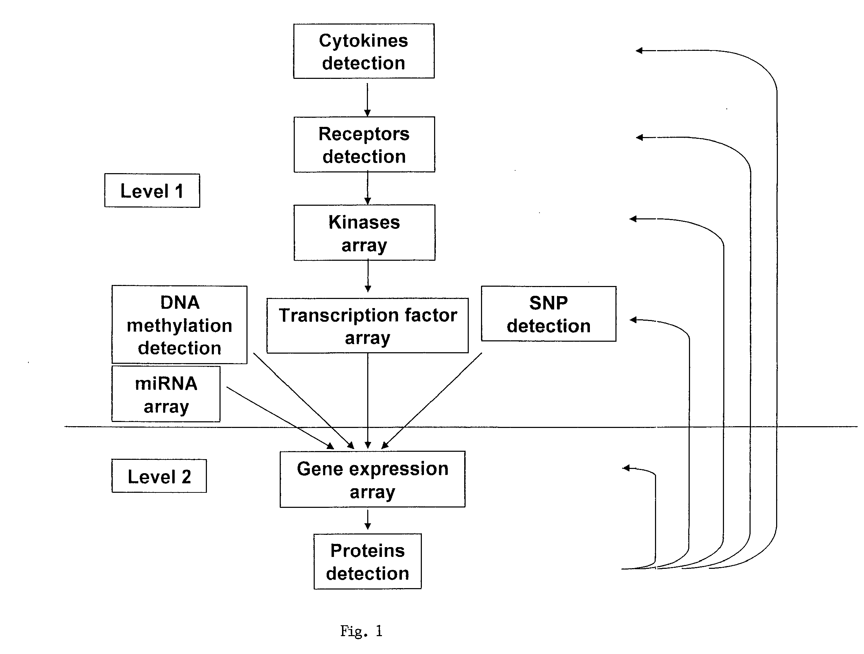

Figure 1 is a schematic representation of a preferred method of the invention for the detection of hierarchical (regulated) changes of a cell in response to an external stimulus and by integrating a least a first level of cell function and a second level of cell function, the molecules involved in the first level (cytokines, receptors, kinases, transcription factors, miRNA) being regulatory molecules interacting with the expression of the molecules of the second level (gene expression, proteins).

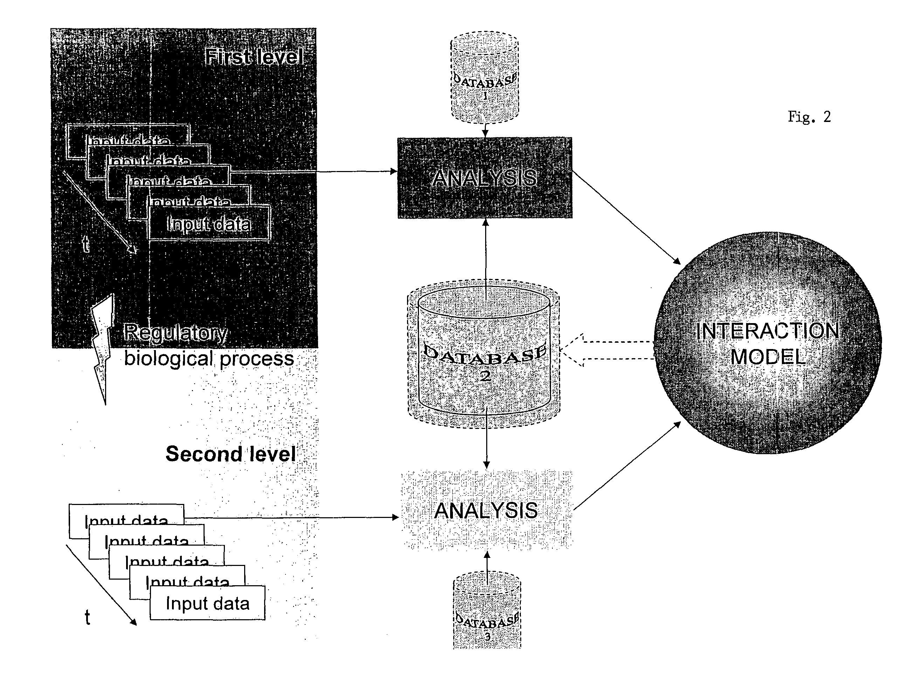

Figure 2 depicts a flowchart of the optionally predictive method performed by an executable program: input of data from 2 levels of cell functions, first one being a regulatory biological process of the second level. First step comprises independent analysis of the input data and optional input from database 1 for first level data and optional input from database 3 for second level data. Input data may be generated from different time periods. These optional database input are pathways, genes annotations, genes networks. In a second step a model response is constructed as a representation of interactions between the 2 levels using optional complementary information drawn from database 2 containing for example validated relational models. Resulting constructed model feeds Database 3 for further predictive analysis.

Figure 3 shows the profile of activated transcription factors in U937 cells stimulated with interferon γ after 15 min of activation (A) and 24h (B) as described in example 2. Unstimulated cells are used as control. The assay was performed on a TF chips capable of detection of 8 different Transcriptional factors.

Figure 4 shows the profile of activated transcription factors in U937 cells stimulated with interferon α (A) and γ (B) after 24h activation as described in example 3. Unstimulated cells are used as control.

Figure 5 shows the profile of activated transcription factors in MCF7 cells stimulated with H₂O₂ after 3 h of activation (A) and 24h (B) as described in example 4. Unstimulated cells are used as control.
Figure 6 shows the profile of activated transcription factors in MCF7 cells stimulated with β-estradiol after 4 h of activation (A) and 24h (B) as described in example 4. Unstimulated cells are used as control.

Table 1 presentation of the gene list which significantly changes with the time frame of stimulation with IFNγ as described in example 2.

Table 2 presentation of the gene list which significantly changes with the stimulation with IFNα and IFNγ as described in example 3.

Table 3 presentation of the gene list which significantly changes with the time frame of stimulation with β-estradiol and H₂O₂ as described in example 4.

### Definition

Unless otherwise defined, all technical and scientific terms used herein have the same meaning as commonly understood by one person ordinary skilled in the art to which this invention belongs.

The terms "biological, physical and/or chemical stimulus" means any parameter (pH, t°, pressure, salinity, UV or ion beam radiation,...), addition or suppression to the cell of any compound (ion, metal, protein, microorganism, drug,...) that may modify the cell characteristics, or cell modification over time (ageing, stress, necrosis, apoptosis, cell transformation or cancerous cell, (stem) cell differentiation).

The terms "expressed genes" are those regions of the genomic DNA which are transcribed into mRNA and optionally then translated thereafter into (poly-)peptides or proteins.

The terms "nucleic acid, array, target nucleic acid, bind substantially, hybridizing specifically to, background, quantifying" are as described in WO97/27317, which is incorporated herein by way of reference.

"Micro-arrays and arrays" mean supports on which single capture molecules or capture probes species are immobilized in order to be able to bind to the given specific protein or target. The most common arrays are composed of single capture probes species being present in predetermined locations of a support being or not a substrate for their binding. The array is preferentially composed of spots of capture molecules deposited at a given location on the surface or within the support or on the substrate covering the support. The capture probes are present on the support in various forms being but not limited to spots. One particular form of application of array is the presence of capture probes in wells having either one of several different capture probes per well and being part of the same support. In one particular application of this invention, arrays of capture molecules are also provided on different supports as long as the different supports contain specific capture molecules and may be distinguished from each other in order to be able to quantify the specific target. This can be achieved by using a mixture of beads having particular features and being able to be recognized from each other in order to quantify the bound molecules.

The terms "capture probe" relates to a molecule capable to specifically bind to a given polynucleotide or polypeptide. Polynucleotide binding is obtained through base pairing between two polynucleotides, one being the immobilized capture probe and the other one the target to be detected.

Capture probes for transcriptional factors also include nucleotide sequences to which the transcriptional factors will bind.

The term "single capture probe species" is a composition of related polynucleotides or polypeptides for the detection of a given sequence by base pairing hybridization or by molecular recognition between polypeptides or proteins. Polynucleotides or polypeptides are synthesized either chemically or enzymatically or purified from samples but the synthesis or purification is not always perfect and the capture molecule is contaminated by other related molecules like shorter polynucleotides or other polypeptides. The essential characteristic of one capture species for the invention is that the overall species can be used for capture of a given target molecule being another polynucleotide or polypeptide sequence.

As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, or pieces of them, are all derived from the mRNA transcript; the detection of such derived products is indicative of the presence and/or abundance of the original transcript in a sample. Thus, suitable samples include, but are not limited to, mRNA transcripts of the genes, cDNA reverse transcribed from the mRNA, cRNA transcribed from the cDNA, DNA amplified from the genes, RNA transcribed from amplified DNA, and the like or pieces of them.

The "hybridized nucleic acids" are typically detected by detecting one or more "labels" attached to the sample nucleic acids. The labels may be incorporated by any of a number of means well known to those skilled in the art, such as detailed in WO 99/32660, which is incorporated herein by way of reference.

Transcriptional factors are proteins which bind to specific sequences of double stranded DNA, called consensus sequences, and when activated either by themselves or with the help of other proteins or enzymes will modulate, either activate or repress, the transcription of the DNA. They are composed of a DNA binding domain and a transactivating domain responsible for their activity and they are part of a large protein complex which interacts with the transcriptional machinery for regulating its activity. Some Transcriptional factors are able to bind single stranded DNA as for example the class of single-stranded DNA binding transcription factors belonging to the Y-box family of protein which influence transcription of both cellular and viral genes (Swamynathan, S. K., Nambiar, A., Guntaka, R. V. FASEB J. 12, 515-522 (1998).

"Protein phosphorylation" refers to post-translational protein phosphorylation. "Phosphorylation" includes phosphorylation on a protein residue being either a tyrosine, serine, threonine and/or histidine. Antibodies that can be used to detect these modifications include phosphotyrosine-specific antibody, phosphoserine-specific antibody, and phospho-threonine-proline antibody, for example. Antibodies that may be used to detect these modifications also include antibodies specific to (a) phosphorylated residue(s) of a protein, or a fragment of the protein containing the phosphorylated residue(s).

As used herein, the term "receptor" refers to a protein associated with a cellular membrane and binding to one or more specific molecules (or ligand) only. Receptors may be naturally-occurring or synthetic molecules. Based on their signal transduction mechanisms they may be classified in four groups: (i) receptors that are ligand-gated ion channels (LGIC), e.g., the nicotinic acetylcholine and gamma-aminobutyric acid (GABA) receptors; (ii) receptors that are associated with an enzymatic activity, e.g. the insulin receptor (a tyrosine kinase) or the atrial natriuretic peptide receptor, (a form of a guanylate cyclase); (iii) receptors, that couple to guanosine triphosphate (GTP)-binding proteins (GPCR receptors), e.g., muscarinic acetylcholine receptors; and (iv) receptors with unknown signal transduction mechanisms (e.g., the sigma receptor).

The term "miRNA" is a non coding small RNA produced by a DICR enzyme from a double stranded RNA precursor. The precursor has a stem loop or hair-pin structure. miRNA are present in animals or plants. They are mainly bound to a protein complex termed miRISCs. They represent one of the components of the RNAi beside other ones like the siRNA. miRNAs regulate mRNA translation whereas siRNAs direct RNA destruction via the RNA interference pathway. miRNAs are processed through at least two sequential steps; generation of 70 nucleotides (pre-miRNAs) from the longer transcripts (termed pri-miRNAs) and processing of pre-miRNAs into mature miRNAs (Lee et al. Embo J. 21 (2002), 4663-4670). miRNAs are typically 20-22 nucleotides non coding RNA that regulate expression of mRNA exhibiting sequences complementary thereto. They are numerous and widespread among eukaryotes, being conserved throughout evolution. The actual known number of miRNA is around 250 for human cells.

The term "DNA methylation" refers to a phenomenon occurring on the cytosine bases from DNA. Cytosine exists in a "normal" and in a methylated version (e.g. with a methyl group attached), but only when directly followed by the base guanine. The consequences of methylation lie in the regulation of gene expression: methylated cytosines in the promoter region of a gene lead to inactivation, thus acting as an "on" and "off" switch for genes. This is a naturally occurring mechanism to prevent all genes in a tissue/cell to be expressed at a time.

The term "Chromatin immunoprecipitation" (ChiP) refers to a method allowing the identification of targets of transcriptional factors. Cells are fixed to crosslink transcriptional factor proteins to the DNA fragments that they bind in vivo. Cells are lysed, the chromatin is sheared, and the transcriptional factor with its associated DNA is immunoprecipitated. The bound DNA fragments are then recovered, PCR amplified and hybridized on arrays harboring the regulatory regions of the questioned genome. DNA fragments protected by chromatin will give a signal on spots containing sequences complementary to the protected region.

The term "SNP detection" refers to a single nucleotide polymorphism. Correlating the effect of particular single nucleotide polymorphisms (SNP) in DNA sequences on the biology of the system can be obtained on microarray. The array can further provide specific capture probes for detecting particular SNP in specific promoter or genes which may allow understanding of the impact of SNP on the biology of the system.

As used herein, the term "assessing" is intended to include quantitative and qualitative determination in the sense of obtaining an absolute value for the amount or concentration of the analyte present in the sample, and also of obtaining an index, ratio, percentage, visual and/or other value indicative of the level of analyte in the sample. Assessment may be direct or indirect and the chemical species actually detected need not of course be the analyte itself but may for example be a derivative thereof or some further substance.

"Biological sample" includes a biological fluid or a biological tissue or an extract thereof. Examples of biological fluids include urine, blood, plasma, serum, saliva, semen, stool, sputum, cerebral spinal fluid, tears, mucus, amniotic fluid, cell cultured or the like. Biological tissues are aggregates of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s).

The term "cell" means any prokaryote or eukaryote cell, preferably a mammal cell, more preferably a human cell which is able to react to an external biological, physical or chemical stimulus. Such cell could be also a tumor cell or a stem cell. The detection and/or quantification of hierarchical (regulated) molecular change of a cell in response to an external biological, physical and/or chemical stimulus is preferably compared with a control cell (or normal cell), which has not been submitted to this stimulus. The characteristics of a cancer cell or of a stem cell could be also compared to a normal cell or to a differentiated cell.

Cell extracts are cytoplasmic and nucleus elements of a cell submitted to membrane lysis by a chemical or a mechanical interaction.

"A control (led) biological sample" is any biological sample taken as a reference compared to a "test biological sample". The control biological sample can be derived from a normal tissue and be compared to the test biological sample being for example a diseased tissue. Diseased tissue refers to a pathological condition in an organism resulting e.g., from infection or genetic defect, and is characterized by identifiable symptoms. The control and the test biological sample can be the same tissue or derived from different organisms, or from the same tissue at different developmental or differentiation stages. The control biological sample can also correspond to untreated cells and the test biological sample to treated cells, wherein the treatment is physical, chemical, physiological or drug administration.

The term "drug" as used herein, should be interpreted broadly to include compounds of known efficacy and safety, drug candidates picked randomly from libraries of compounds or drugs at every levels of investigation.

Drugs include compounds which affect the level of activation of cells and could be modulators of kinase/phosphatase activities.

### Detailed description of the invention

The method of the invention is performed on cell extract being prepared and handle according to the nature of the cell component to be assayed. The cells tissues or their extracts are separated into 2 or more samples which are then processed as described here below and in the example in order to perform the different specific assays of levels 1 and 2 as proposed in the invention. Thereafter, the data (or results) of the different assays (detection and/or quantification) are re-associated and processed (collected, correlated and integrated as models) according to the invention for characterizing the hierarchical molecular change in the cell.

The assay comprises a control condition which differs from the analysed sample in that, in the sample, the cells have been exposed to a stimulus, which may be a physiological, pathological, mechanical, chemical, toxic, pharmaceutical stress or temperature. One particular stimulus is the binding of one or more ligand to the receptor(s) of the cell. The detection and/or quantification of hierarchical (regulated) molecular change of a cell in response to an external biological, physical and/or chemical stimulus is preferably obtained by comparing the detection and/or quantification of the cell response to a detection and/or quantification of a control(led) cell which has not been submitted to the stimulus. The biological test sample and the control experimental conditions are preferably analyzed in assays performed on the same support. In the second step of the invention method, the assay are capable of detection of at least 5 and preferably at least 10 different transcriptional factors and/or MAP-kinases but the significant changes observed in the activities in a special cell condition refer sometimes to only one or a few (less than 5) of transcriptional factors and/or MAP-kinases.

The arrays to be used are any conventional "biochip structure" on which corresponding biological samples are incubated. These arrays comprise as capture probes polynucleotide sequences and/or peptide sequences.

Advantageously, each of the different assays is performed on a sample containing less than 100 mg and better less than 10 and even less than 1 mg protein.

The assays, as required by the invention, are preferably performed on the same cell extract incubated in separated assays being physically separated. In the preferred embodiment the different assays are performed on arrays being present in different supports or on the same support.

Furthermore, the step of detecting and optionally quantifying for gene expression in the sample or sample extract by the hybridization of polynucleotides on the array is preferably performed on a single capture nucleotide specie used as capture probe.

Advantageously, the expressed genes or proteins detected and/or quantified are selected among a multiplicity of at least 100, preferably at least 200, more preferably at least 1000 genes or proteins, encoded in the genome of the cell.

More specifically, the detection and/or quantification of the expressed genes is preferably obtained by the steps of:
a) obtaining (providing) a cell extract containing a pool of target nucleic acids comprising RNA transcripts of one or more of the said genes, or nucleic acids derived therefrom using said RNA transcripts as templates;
b) hybridizing said pool of target nucleic acids to a microarray of polynucleotide capture probes immobilized on a solid support surface, wherein each different polynucleotide probe is localized at a predetermined location of the said solid support surface and wherein each polynucleotide probe is specific of a target nucleic acid and,
c) detecting and/or quantifying the hybridization of the said nucleic acids to said polynucleotide probes of the microarray and wherein the quantification is proportional to the expression level of the said genes.

In a specific application, the analysis of the transcriptome functions is performed on the DualChip human general as proposed in US 2004/0229225 and on the DualChip breast cancer as proposed in US 2004/0191783. The DualChip human general allows the simultaneous analysis of 202 different genes belonging to 13 vital functions. Each gene detection is performed in triplicates. The value for the presence of each gene is the average of the three values and the mean is calculated together with the standard deviation. Each of the value is then corrected using internal standards which have been added in the analysis in a given concentration. Thereafter, a correction for house keeping genes is also performed as an option for variations within the arrays. This process gives absolute values for the genes present in a test experiment compared to a control condition or to reference sample. Example of such micrarray is presented in example 1.

In a preferred embodiment, the quantitative determination of the expression of the multiplicity of genes is performed on genes belonging to or being representative of at least 9 of the vital cellular functions selected from the group consisting of: apoptosis, cell adhesion, cell cycle, growth factors and cytokines, cell signalling, chromosomal processing, DNA repair/synthesis, intermediate metabolism, extracellular matrix, cell structure, protein metabolism, oxidative metabolism, transcription, and house keeping genes, said functions being represented by at least 4 different genes.

Advantageously, the transcriptional profile is provided in the following way. The transcriptional profiling genes are divided into 3 categories being but not limited to the over expressed, repressed and non significantly change genes when compared the cell in the studied sample or situation and the reference sample.

Preferably the assay for TF is performed according to US 2004/0185497 and US 2003/0162211. The detection of transcriptional factors or proteins able to bind DNA sequence present in cell lysate (including nuclear lysate) or present in a body fluid has to be very sensitive with less than about 10-12 mole of transcriptional factor to be detected in 50 micro-liters in an assay and better having a limit of detection lower than 10-14 mole in the assay. Sensitivity of the TF assay has to be sufficient for the detection of transcriptional factors naturally present in cell lysate.

The method also preferably comprises the step of identification of at least one characteristic specific of a given activated transcriptional factor. Some transcriptional factors bind to their consensus DNA sequence without activating the transcriptional machinery. The activation is then associated with one or several specific changes, the most common one being the phosphorylation (or dephosphorylation) at specific amino acid(s) of the protein. An example of such transcriptional factor is CREB, which is only active when phosphorylated.

According to a preferred embodiment, binding of the TF is followed by incubation (and washing) with labelled compounds able to react with said transcriptional factor or with first compound already bound to said TF, preferably (monoclonal) antibodies directed against the anti-transcriptional factor antibodies or specific hypervariable portions thereof (Fab', Fab2', etc.). Said last antibodies are preferably labelled with non radioactive markers allowing a detection, preferably by colorimetry, fluorescence, bioluminescence, electroluminescence or precipitation of a metal deposit (such as silver staining as proposed by EP1179180). Other secondary binding proteins like protein A which bind to antibodies are also an embodiment of the method. Said non radioactive test is preferably based upon a colorimetric assay resulting from a catalytic activity such as enzymatic or chemical reduction of silver cation. If direct method such as mass spectrum analysis is sensitive enough it can be used for direct detection of the bound factor.

The method of the invention can also differentiate the DNA binding of the transcription factor and its activity. This activity is associated with one or several particular characteristics of the factors, the most common one being the phosphorylation at specific locations or the dissociation of inhibitory proteins. By using appropriate antibodies directed against these elements, it is possible to determine the amount of transcriptional factors in their active form. As an example, CREB can bind to its DNA consensus sequence without being active. Its activation results from a specific phosphorylation at a serine group, making the P-CREB. The preferred embodiment of this invention is to perform assay for TF on arrays.

The arrays or similar technology allows the simultaneous detection of different factors present in the same sample, with factors binding to their consensus sequence in the same reacting conditions. Solutions are available for example from Promega (Madison, Wis., USA) for the binding of several factors (Catalogue E3581). Conditions which allow the binding of the factors studied may be optimized by the person skilled in the art.

In particular, the detection and/or the quantification of the different (activated) transcriptional factors is performed by the steps of:
a) obtaining (providing) a cell extract containing (activated) transcriptional factors;
b) contacting the cell extract under conditions allowing the binding of (activated) transcriptional factors with a microarray made of at least 5 capture probes/cm² of the solid support surface, wherein each different capture probe is localized in a predetermined location of the said solid support surface, wherein each probe is specific of an (activated) transcriptional factor and,
c) detecting and/or quantifying signal(s) resulting from the binding of the (activated) transcriptional factors to their corresponding capture probes, and wherein the location of the signal is related to the transcriptional factor identity present in said location.

Preferably, the capture probes for the TF assay are double-stranded DNA sequences immobilized on the solid support surface at a concentration of at least 0.01 micromoles/cm², and these double-stranded DNA sequences comprise a specific sequence able to bind specifically an activated transcriptional factor and wherein the double-stranded DNA sequences are linked to the solid support surface by a spacer having a length of at least 6.8 nm.

More preferably, these double-stranded DNA sequences comprise a spacer of at least about 13.5 nm between the specific sequence and the surface of the solid support. In another preferred embodiment, the capture probes are antibodies bound to the support.

In a particular embodiment, the assay includes the assay of the activity of kinases preferably the MAPKinases involved in cell regulation or activation pathways.

More particularly, activities of the kinases being part of the MAP-kinase activation pathways are determined by measurement of the level of phosphorylation of the proteins, enzymes being the substrates of the kinases and phosphatases being part of the pathways or of synthetic peptides in an in vitro assay. The activity can be obtained by calculating the ratio between the level of specific phosphorylation to non-phosphorylated form or to the total enzyme content. The degree of activation of the enzymes is obtained preferbaly by quantification on the same support, preferably in the form of arrays of the amount of phosphorylated enzymes compared to the total amount of enzymes obtained on a second array incubated with the same cell extract.

Quantitative determination of the different activated MAP-kinases is preferably performed by the steps of:
a) obtaining (providing) a cell extract containing activated MAP-kinases and phosphatases;
b) determining the equilibrium between the activities of kinase/phosphatase enzymes on proteins participating in signal transduction into cells or by quantitatively determining the level of phosphorylation of cellular proteins participating in signal transduction belonging to a cascade of phosphorylation leading to the activation of at least one transcriptional factor

In a preferred embodiment of the invention, the assay for the kinase activity is realized according to the method described in US 2004/0265938.

In another embodiment, the invention assays for different kinase cascades and the resulting phosphoproteins. Protein phosphorylation occurs on an amino acid residue selected from the group consisting of tyrosine, serine, threonine and histidine.

In still another embodiment, the activities of specific kinase/phosphatase enzymes are determined, which give an overview of the cell's present activation by detection and/or the quantitative phosphorylation level of a variety of different cellular targets of kinases/phosphatases, resulting from the equilibrium between these kinases/phosphatases as provided in US 2004/0265938.

The ratios of phosphorylated level of proteins can be compared to samples of cells being either not or sufficiently activated, so that the experimental values of the ratio provide an evaluation of the cell activation compared to the two extreme cell situations.

The same sample is preferably incubated on two different arrays, the first being used for the quantification of the total proteins captured and the second for the quantification of the phosphorylated proteins, with the ratio between the two quantifications giving the level of activation of the cells. The two arrays may contain a plurality of identical capture molecules for the target proteins to be detected, and the level of target proteins is quantified on one array using detection molecules being specific for each of the proteins and the level of phosphorylation of target proteins is quantified on the other array using detection molecules being specific for the phospho-proteins.

The assay preferably includes one or more of the serine, threonine kinases among but and not limited to: MKK1, MKK2, MKK3, MKK3, MKK4, MKK5, MKK6, MKK7, MEKK1, MEKK2, MEKK3, MEKK4, TPL2, ASK1, CK1α, CK1α_like, CK1ε,CK1δ, CK1γ1, CK1γ2, CK1γ3, PAK1, PAK2, PAK3, PAK4, PAK5, PAK6, CASK, CAMKIIβ, CAMKγ, CAMK2A, CAMKIIδ, CAMKIIδ_like, AMPK1_like, AMPKA1, PRKK_like, SNF1_like, STK29, MARK, MARK3, EMK, CHK1, PIM1, PIM2, HUNK, STK33, PKCµ, PKCν, PKD2, CHK2, DMPK, DMPK_like, CDC42BPB, ROCK1, ROCK2, KPM, WARTS, SAST, PKN, PDK1, STK33, TSF1, GAK, IKKe, RAF1, RAF1_like, RIPK1, RIPK3, TAK1, TESK1, TESK2, WNK1, WNK2, WNK3, STK2, NEK3, NEK6, MINK1, MINK2, SPAK, IRE1, IRE1B, PRKR, TPL2, WEE1_like, TOPK, HIPK2, YAK1, GSK3b, DYRK1A, DYRK1B, DYRK2, DYRK3, DYRK4, LCK2, STK9, MOK, CDK8, MSSK1, SRPK1, GLK, TLK1, TLK2, PKL, SAK, PIM1, PIM2, CAMKK, AMPKa1, SNF_like2, MELK, EMK, MYLK, DRAK1;
and the tyrosine kinases not limited to: CDK210, NKLIAMRE, STK9, P38, P38b, P38g, P38d, ERK1-5, JUNK1-3, JAK1, JAK2, JAK3, TYK2, ZAP70, SYK, LYN, HCK, BLK, LCK, FYN, FGR, SRC, YES1, MATK, CSK, FRK, ARG, ABL, ITK, TEC, BTK, TXK, BMX, FER, FES, TNK1, ACK1, FAK, PYK2;

The external biological stimulus can be a binding of one or more ligands (preferably cytokines) to receptor(s) of the cell.

The molecular change at a first level of the cell function (first assay) also comprises the assay for the detection and/or quantification of at least 5 and preferably 20 different cytokines being present in the sample or cell environment (intracellular or extracellular).
More specifically the detection and/or quantification of the cytokines is performed by (the first assay comprises) the steps of:
(a)obtaining (providing) a cell extract containing a pool of cytokines;
(b)contacting the cell extract under conditions allowing the binding of cytokines with a microarray comprising at least 5 and preferably 20 different capture probes being antibodies immobilised on a solid support surface, wherein each antibody is localised at a predetermined location of the surface wherein each antibody is specific for a corresponding cytokine;
(c)detecting and/or quantifying signals resulting from the binding of the cytokines to the capture probes wherein the location of the signal is related to the cytokine identity (detection and/or quantification) which is present in the said location.

Cytokines are generated by different cells, they act autocrine and paracrine and they may behave pleiotropic as well as redundant and antagonistic. Their ability to control and regulate cell differentiation and growth requires recognition and binding to specific receptors. These receptors are part of cell surface and mediate the cytokine binding event into cytoplasmic signals. Alternate pathways might be triggered by cytokines, as for example the Ras-MEK-ERK signal pathway, also know as MAP kinase signal transduction cascade. The cytokines are responsible for activation or repression of cell activation.

The different cytokines are preferably assayed by ELISA or on an array by detecting a signal present on specific locations on the array preferably bearing specific antibodies as capture probes, said signal at one location being related to the presence of one cytokine with the detection of at least 3, preferably at least 5 , more preferably at least 10 and even more preferably at least 20 cytokines or receptors among the IL-1a, IL-1b IL-1 RA, IL-2, IL-4, IL-6, IL-8, IL-10, IL-12 p40, 12 p70, IL-17, TNF-a, TNF-RI, TNF-RII, IFN-g, GM-CSF , IFN-g, GM-CSF, Eotaxin, MIP-1a, MIP-1b, MCP1 and RANTES. In a particular embodiment, the assay also includes the assay for the presence of cytokines in the sample or cell environment as proposed in WO00127611A2.

The method of the invention can further comprise a receptor activation assay which is preferably performed on a receptor that activates kinases selected from the group consisting of serine, threonine or tyrosine kinase enzymes.

A preferred method of receptor activation assay is a luminescent assay reagent used for the analysis of G-protein coupled receptor signalling as described by Stables et al. (1999, Recept. Signal Transduct Res., 19, 395-410).

In brief, the assay is performed as follows. Several reporter gene assays have been described where gene transcription is activated as a consequence of a specific signal transduction event such as activation of adenylyl cyclase. Reporter genes typically consist of specific responsive elements placed upstream of a minimal promoter, which together control the expression of a readily detectable reporter protein, such as luciferase. The dual glow-signal firefly and Renilla luciferase assay allows the simultaneous measurement of two reporter genes in the same well of a 96-well plate. The assay is used for the simultaneous analysis of agonist activity at two G-protein coupled receptors which signal through activation of the G-protein alpha sub-unit, G alpha S.

The present method also allows the specific determination of the expression status various subtypes and sub-subtype genes of the GPCR or LGIC receptors regulated by all amine neurotransmitters as provided in US 2005/0053946. The method comprises the step of obtaining nucleic acids from a biological sample and contacting the nucleic acids with a micro-array, containing on specific locations thereon at least one capture probe derived from a gene encoding a receptor for a dopamine, a histamine, a serotonin, an adrenergic and a cholinergic neurotransmitter, and determining the expression profile of said receptors in the biological sample, by evaluating the two dimensional pattern of data present as intensities of spots on the surface of the micro-array, one detected and/or quantified spot being sufficient for obtaining the information on one neurotransmitter subtype.

In a preferred embodiment, the micro-array contains capture probes representing genes encoding at least 3 receptors for dopamine, at least 4 for histamine, at least 7 for serotonin, at least 3 for adrenergic and at least 7 for cholinergic neurotransmitters selected among the group consisting of the following subtypes:
dopamine (Drd1a, Drd2, Drd3, Drd4, Drd5), for histamine (Hrh1, Hrh2, Hrh3, Hrh4), for serotonin (Htr1a Htr1b, Htr1d, Htr1e, Htr2a, Htr2b, Htr2c, Htr3A, Htr3B, Htr4, Htr5a, Htr5b, Htr6, Htr7), for adrenergic (ADRA1A, ADRA1B, ADRA1D, ADRA2C, ADRB2), for cholinergic (CHRNA2, CHRNA3, CHRNA4, CHRNA5, CHRNA7, CHRNB1, CHRNB2, CHRNB3, CHRNB4, CHRND, CHRNE, CHRM1, CHRM2, CHRM3, CHRM4, CHRM5) and for the trace amine (TA1, TA2, TA3, TA4, TA6, TA7, TA8, TA9, TA10, TA11, TA12, TA13, TA14, TA15). Some possible subtypes may be commonly detected on the same capture probe.

The invention includes the assays for receptors of tyrosine kinases being not limited to: DDR1, DDR2, ROS, AXL, MER, SKY, MET, RON, RYK, TRKA, TRKB, TRKC, MUSK, CCK4, ALK, ROR1/2, RET, TIE, TEK, FLT3, FGFR1, FGFR2, FGFR3, FGFR4, VEGFR1, VEGFR2, VEGFR3, KIT CSF1R, PDGFRA/B, ERBB2, ERBB3, ERBB4, EGFR, EPHA1, EPHA2, EPHA3, EPHA4, EPHA5, EPHA7, EPHA8, EPHB1, EPHB2, EPHB3, EPHB4, EPHB6, INSR, IGF1R, INSRR, LMR1, LMR2.

In another embodiment, the molecular changes at a first level of the cell function are further determined by submitting the cell extract to an assay (the first assay comprises the steps) for the detection and/or quantification of at least 5 and preferably 50 and even preferably 100 different miRNA or siRNA in the cells.

The method also allows the determination of cellular transcriptional regulation by the simultaneous detection and quantification of multiple miRNAs present in a cell.

The detection and/or quantification of the different miRNA is preferentially performed by (the first assay comprises) the steps of:
a) obtaining (providing) a cell extract containing a pool of miRNA;
b) elongating or copying or ligating said miRNAs into target labeled polynucleotides;
c) hybridizing the said target labelled polynucleotides to a microarray of polynucleotide capture probes immobilized on a solid support surface, wherein each different polynucleotide is localized at a predetermined location of the said solid support surface and wherein each polynucleotide probe is specific for a corresponding target polynucleotide;
d) detecting and/or quantifying signals resulting from the binding of the target labelled polynucleotides to the polynucleotide probes wherein the location of the signal is related to the miRNA identity (detection and/or quantification) present in the said location.

In a preferred embodiment, the array comprises capture probes represented by polynucleotides having a sequence identical to miRNA. The array may contain capture probes being polynucleotides having a sequence complementary to miRNA.

Preferably, the different capture molecules present on the array cover most and preferably all of the miRNA present in a cell. In another preferred embodiment, the capture molecules allow the binding of the miRNA related to the regulation of the gene of interest in the given application.

In principle, the micro-array contains at least 3 capture probes, e.g. one capture probe associated with a miRNA. Yet, the number of capture probes on the micro-array may be selected according to the need of the skilled person and may contain capture probes for the detection of up to about 5000 different miRNA, e.g. about 100, or 200 or 500 or 1000, or 2000 different miRNA involved in the cell transcriptional regulation.

In a preferred embodiment, to unravel the cellular transcriptional regulation, the pattern of at least 3 miRNAs obtained by the method of the invention is correlated with the activation of TF and the pattern of expression of the regulated genes in the same sample.

In another embodiment, the detection and quantification of the miRNA is performed together with the assay on the expression of the genes and the activation of TF through the detection and quantification of mRNA or proteins in the same sample or on extracts coming from the same biological sample.

The method also allows a determination of cellular transcriptional regulation by simultaneous detection and quantification of multiple DNA methylations, Chromatin protections and Single Nucleotide Polymorphism (SNP) sites in DNA in a cell on an array and by detecting signals present on specific locations on the array, said signals at such locations being related to the presence of a methylation, chromatin protected or SNP sites with the detection of at least 5, preferably at least 20, more preferably at least 100 and even more preferably at least 1000 different methylations, chromatin protected or SNP sites on the array being indicative of a given methylation, chromatin protected or SNP sites modulating cellular transcriptional regulation.

In a preferred embodiment the array comprises capture probes represented by polynucleotides having a sequence identical to the methylation, chromatin protected or SNP sites. In another embodiment, the array contains capture probes being polynucleotides having a sequence complementary to methylation, chromatin protected or SNP site.

Preferably, the SNP are assayed using arrays according to the method described in WO0177372. The DNA sequence for which a SNP is to be assayed is first amplified or copied by consensus primer(s) the copied or amplified sequences are hybridized on the array. SNP are also assayed on array bearing core sequence probes as described in US-6,852,488 by comparison of the target binding affinity on the core sequence compared to the single nucleotide variation of the core probe. In another preferred method, the SNP is detected by a reverse chips process as described in WO0196592.

The preferred embodiment for the chromatin protected assay is the following: cells are fixed to crosslink transcriptional factors proteins to the DNA fragments that that they bind in vivo. Cells are lysed, the chromatin is sheared or digested by nucleases, and the transcriptional factors with its associated DNA are immunoprecipitated. The bound DNA fragments are then recovered, PCR amplified and labelled, then hybridized on arrays harbouring the regulatory regions in the genome. DNA fragments protected by chromatin will give a signal on spots containing sequences complementary to the protected regions.

The preferred embodiment for the SNP detection is to provide amplified sequence and to hybridized them immediately on arrays containing single nucleotide difference at the site of the possible mutation. The SNP detection is preferably performed according to the reverse chips technology as described in EP1290228.

Yet, the number of capture probes on the micro-array may be selected according to the need of the skilled person and may contain capture probes for the detection of up to about 5000 different methylation, chromatin protected or SNP sites, e.g. about 5 or 20 or 100 or 1000 different methylation, chromatin protected or SNP site involved in the cell transcriptional regulation.

In a preferred embodiment, the signal present on a specific location on the array corresponds to a pattern of at least 5, 20, 50 and even 100 methylations, chromatin protected or SNP site's.

To unravel the cellular transcriptional regulation, the pattern of at least 5 methylations, chromatin protected or SNP sites obtained by the method of the invention is incorporated into the model and correlated with the activation of TF and the pattern of expression of the regulated genes in the same sample (e.g. provided by a second array). More particularly, the pattern of at least 3 methylations, chromatin protected or SNP sites is incorporated into the model and correlated with the pattern of expression of the methylation, chromatin protected or SNP site target genes in the same sample (e.g. provided by a second array). The pattern of at least 3 methylations, chromatin protected or SNP site's can be correlated with the pattern of expression of genes having mRNA sequences complementary to the corresponding methylation, chromatin protected or SNP site sequence in the same sample (e.g. provided by a second array). The pattern of at least 3 methylations, chromatin protected or SNP site's obtained by the method of the invention can be correlated with activated transcriptional factors in the same sample.

In another embodiment, the detection and quantification of the methylation, chromatin protected or SNP site is performed together with the assay on the expression of the genes and the TF through the detection and quantification of mRNA or proteins in the same sample or on extracts coming from the same biological sample. The methylation, chromatin protected or SNP site and the genes detection assays can be performed on arrays present on the same support. Comparison is performed between the pattern of methylation, chromatin protected or SNP site present in the sample with the pattern of genes differentially expressed in the same sample and the activated or inactivated TF. Preferably, the arrays are present on different supports.

The level of genes is then related to the presence and amount or identity of the corresponding methylation, chromatin protected or SNP site and to the presence of activated or inactivated TF.

The method according to the invention further comprises a receptor activation assay, preferably a receptor activation assay of a receptor that activates kinases selected from the group consisting of serine, threonine or tyrosine kinase enzymes.

According to a preferred embodiment of the present invention, the first assay of the method further comprises a detection and/or a quantification of at least 5, preferably at least 20 different methylation sites of a DNA sequence, a detection and/or a quantification of chromatin protection assay and/or a detection and/or a quantification of at least 5, preferably at least 20 different signal nucleotide polymorphism sites of a DNA sequence.

Preferably the assay for the detection and/or quantification of proteins encoded by the expressed genes is assayed on a microarray of capture probes immobilized on a solid support surface and wherein the detection and/or quantification of the proteins is determined by a signal resulting from one characteristic specific of the proteins and wherein the signal is quantified. The binding of the protein on the surface of the solid support can be obtained through an epitope of the protein which is recognized by a capture probe being an antibody.

In another embodiment, the signal resulting from one characteristic specific of the proteins is the binding of an antibody against an epitope of the protein, or the proteins are identified by determination of their specific molecular weight, preferably using a MALDI mass spectrometer.

In a particular embodiment, the proteins are digested into peptides before their detection and/or quantification by the mass spectrometer analysis. The identity of the protein is determined from the identification of one or several specific peptides or by the determination of the sequence of one or several of the peptides.

The detection and/or quantification of at least 10 different proteins can be also performed by the proteomic analysis using a 2D gel separation by electrophoresis. The separation is performed according to the molecular weight in one direction and to the isoelectric point in the other direction. The identification of the protein is obtained according to their position in the gel which is dependant on their physical character as the molecular weight and isoelectric point. Quantification is performed by quantification of the spots present on the gel after staining of the gel using colour or fluorescent dyes such as but not limited to Cy3 or Cy5. Proteins are also preferably identified by reaction with specific antibodies after transfer of the protein on a membrane. The proteins are preferably detected and/or quantified after their deposit in the form of array, by Mass spectrometer either by MALDI-MS or MALDI-MS-MS analysis. The proteins are digested by enzymatic digestion and the peptides analyzed and identified by mass spectrometry. Quantification is preferably obtained by the determination of the intensity of the mass spectrum signal using a reference protein as standard.

As represented on the fig. 2, the invention provides a computer system for integrating and correlating the results of detection and/or quantification of hierarchical (regulated) molecular change of a cell in response to an external biological, physical and/or chemical stimulus, and comprising a memory unit, a central processor unit and a storage media containing an executable program performing the steps e), f), g) and possibly d) of the method according to the invention and comprising the sub steps of receiving input of data (results) of a first and second level of a cell function, constructing from these input data a model response as a representation of interactions between the 2 levels of cell function, wherein said model highlights the regulatory biological process on the second level of the cell function (model of interactions between molecules of the first and the second level).

The computer system can be an Intel Pentium based processor of 166MHz or more, with a memory unit of 32MB or more of main memory. The storage media can be for example hard drives, optical drives (CD-ROM, DVD-ROM, DVD-RAM), USB flash medium, DAT tape, ZIP drive, floppy disks of various formats 3.5 inches or 5.25 inches, an Ethernet virtual drive, directly mounted to the computer or remotely connected to said computer system.

The results of detection and/or quantification of the molecules are preferably digitalized images of microarrays, various raw data extracts from the digitalized images of microarrays (which allow a relative quantification of the molecules detected on these microarrays) or are molecules (transcriptional factors, MAP-kinases, genes or proteins) relative abundance or profiles. The analysis of the input data of the first level and/or second level is preferentially supplemented by a database input providing relational information comprising pathways, networks, annotations obtained and collected from scientific papers or literature.

Furthermore, these input data (results) coming from the first and second levels may be enhanced by an additional database comprising validated interaction models between said two levels, previous computed pathway mapping on said regulatory biological processes, said database being grown up by backward-feeding of computed interaction models from previous experiments.

In a preferred embodiment, the input of data of the first level of cell function comprises transcriptional factor data related to the activation status of transcriptional factors when comparing the cell in a studied cell extract or situation and a reference cell extract, these data (collected in a database of the computer system) comprising: digitalized image intensities from microarrays, raw data extracted from said images, transcription profile computed from said raw data. The input data (results) of the first level also preferably comprises miRNA relative abundance data and/or activated MAP-Kinase data and/or cytokine data and/or SNP sites and/or methylation sites identification.

In a preferred embodiment, the input of data (results) of the second level of cell function comprises gene expression data related to the relative abundance of expressed genes (when comparing the cell in the studied cell extract or situation and the reference cell extract, not submitted to the tested stimulus), these data comprising: digitalized image intensities from microarrays, raw data extracted from said images, gene expression profile taking the form of ratio of expression computed from said raw data and pathway response formed based on said ratio. The input data (results) of the second level also preferably comprises synthesized proteins (profiles) relative abundance data.

This executable program acquires these input data from a database located on the storage media using a standard computer language for accessing and manipulating databases, for example SQL (Structured Query Language).

The executable program constructs a hierarchical model of the effect of the regulatory biological process on the second level by using but not limited, directed network models, K-means, hierarchical clustering, self-organizing maps, neural networks, Bayesian networks, Gaussian graphical models, co-expression networks using conditional independence, graph theory networks.

The model is preferably built by creating a relational network made up of nodes representing entities from said levels and directed or undirected links between said nodes evaluating biological interactions.

The relational network is characterized as a random network, scale-free network or hierarchical network depending on the organisation of the nodes and links forming the network. The organisation is inferred from the degree distribution of the nodes defined as for example the probability distribution of the number of links of a single node.

The nodes are preferably clustered in subset of nodes connected in a specific wiring diagram such as square lattices, triangles, pentagons. Specific subsets occurring more frequently than randomized ones (designated as motifs) being elementary units of biological networks providing information about the typical local interconnection patterns in said network.

These motifs can be correlated with an alternate database comprising pathways, networks, annotations obtained (collected) from scientific papers or literature.

The executable program may construct an interaction model between the 2 levels by mapping the pathway response obtained from gene expression and/or protein input data to the regulatory biological process.

The executable program matches in a first step transcription factor activation profile of the input data to the promoter sequences of regulated genes contained into a database located in said storage media by, but not limited to, SQL queries, and in a second step correlates these information with gene expression input data. Consensus sequences of the different Transcriptional factors are found on the link:
http://molsun1.cbrc.aist.go.jp/research/db/TFSEARCH.html.
Sequence of the promotors or the genes are part of the genome sequence and are found in data bases such as but not limited to http://www.ncbi.nlm.nih.gov/BLAST/.

In a preferred embodiment, the executable program matches in a first step gene expression input data to the promoter sequences of regulated genes contained into a database located in the storage media by but not limited to SQL queries, and in a second step correlates these information with transcription factor activation profile of the input data.

The program constructs a global model of interactions between the 2 levels of cell function by processing the input data at once and for example but not limited to computing scores of correlation between the regulatory biological process and the second level.

The data (results) obtained from the level 1 and 2 are advantageously expressed over a the time frame variable. The time frame variable are preferably the time period over which the experiment has been performed and for which data have been obtained at (after) different periods of time. A delay parameter can be introduced to account for response delays between the different levels (layers) of response. Time frame variable provide additional information or possible interactions (links) between molecules of the first level and molecules of the second level, especially for the regulatory process and the feed back going on in the cells considered as a highly regulated system.

In the present invention, the step e) of the method (correlating the results of detection and/or quantification of the expressed genes and/or synthezised proteins to the results of detection and/or quantification of the activated transcriptional factors and/or activated MAP-kinases), the step f) of the method (integrating the results of said detection and/or quantification and their correlation model of a hierarchical system, preferably by a computer) and the step g) of the method (characterizing, (preferably by a computer) the hierarchical molecular change in the cell resulting from the external biological, physical and/or chemical stimulus) are obtained preferably by the steps of:
- the obtained results of detection and/or quantification are compared with a database comprising input providing relation information between molecules (especially pathway network or annotations of these molecules present in the patents and scientific papers). Examples of such pathway network or annotations are known binding properties of molecules, known activation binding properties of molecules, presence of specific portions in the sequences of nucleotide sequences which could be bound by a specific activating molecules, such as transcriptional factors;
- the obtained results of detection and/or quantification are compared to known levels of expression of genes or proteins.

The model obtained is completed with additional information present in databases comprising validated interactions models between the two levels of the cell function and previous computed pathways mapping said interactions in a general regulatory biological process.

Preferably, the model is a graphical model comprising various relational networks made of nodes representing entities of said level (detected and/or quantified molecules) and directed or undirected links between said nodes.

Preferably, said relational network comprises random networks, scale-free networks or hierarchical networks depending on the organization of these nodes and links, and the obtained results.

More preferably, in the models according to the invention, the nodes are clustered in a subset of nodes connected on a specific diagram, represented by squares, triangles or polygons. Furthermore, these nodes could be annotated with the specific characteristics of the detected and/or quantified molecules.

In particular, these characteristics are related to their quantification results, their presence in specific compartments of the cell (nucleus, membranes, cloroplasts, etc).

In the preferred embodiment, the model is constructed from the data resulting from the detection and/or quantification of molecules (expressed genes, synthesized proteins, transcriptional factors, activated MAP-kinases, miRNAs and cytokines) relative abundance values or profiles.

In a particular embodiment the data are the results of detection and/or quantification from the analysis of the cell extracts obtained on 4 arrays comprising at least an array on different expressed genes and/or proteins and at least one array on different activated transcriptional factor and/or different activated MAP-kinases and at least two arrays on miRNA and/or different methylation sites and/or SNP sites and/or cytokines.

In another embodiment the data are the results of detection and/or quantification from the analysis of the cell extracts obtained on 5 arrays comprising at least an array on different expressed genes and/or protein and at least an array on different activated transcriptional factor and/or different activated MAP-kinases and at least two arrays on miRNA and/or different methylation sites and/or SNP sites and/or cytokines.

In one embodiment, a relationship (link) is made between the MAPK activation and the TF activation with the transcriptional profiling of the cell (possibly collected in a database connected to a computer).

In another embodiment, the cellular regulation pattern considered as a hierarchical system, includes in the model, the information given by the presence of specific cytokines which pathways of activation through the binding to specific receptor is included in the overall description of the kinases: phosphatases activation which in turn act on the TF and finally explain the changes obtained in the gene expression and the protein synthesis.

In still another embodiment, the model incorporated the link between the presence of cytokines, the activation of the MAPK and/or the TF activation and/or the profiling of the miRNA and the transcriptional profiling.

In another embodiment, the invention include in its model the relationships between the the amount of specific miRNA, the presence of activated TF and the transcriptional profiling of the cell.

In another embodiment, the method of the invention incorporates into the model (from databases) the activation of specific receptors in the presence of drugs, ligands, agonists or antagonists of the receptors.

In one embodiment, the invention provides information on the cell being considered as a system biology.

Alternatively, the method and the kit of the present invention may be used for detecting and/or quantifying hierarchical (regulated) molecular change of a cell in response to an external biological, physical and/or chemical stimulus. Proceeding accordingly allow to e.g. elucidate the role of particular genes in a given physiological event or pathological situation, such as stress, ageing, stem cell differentiation, haematopoiesis, neuronal functional status, diabetes, obesity, transformation process such as carcinogenesis, protein turnover or circulatory disorders as atherosclerosis infection, neoplasm (neoplasia), cancer, an immune system disease or disorder, a metabolism disease or disorder, a muscle or bone disease or disorder, a nervous system disease or disorder, a signal disease or disorder, or a transporter disease or disorder.

According to a particular embodiment, the invention allows the identification of a key regulatory element responsible of the main molecular changes occurring in the system under a particular stimulus. The change into a key element would mimick the main changes observed under a given stimulus. In particular the invention is performed for modelling the effect of a change occurring in a particular cell component being part of the regulatory system. The invention also predicts the effect on the overall hierarchical model of change in the activity of one of the molecular component being part of the model. Change in a molecular component include but are not limited to the inhibition or activation of enzyme including kinase and phosphatase, of transcriptional factor, of ligand being cytokine or agonist or antagonist being bound to their receptor, of protein/protein interaction forming active or inactive complex, of DNA methylation or acetylation, of the presence of gene specific miRNA or other regulatory mechanism.

In one embodiment of this invention, the model allows the prediction of the response of the model to perturbation and the possible engineering for industrial or medical purpose. The prediction is then part of the synthetic biology as proposed by Arkin, A. P. 2001 (Sciencedirect 12, 638-644) and allows determining potential targets for drug development. In a preferred embodiment, the assay is used for the determination of potential target for drug development or in synthetic biology.

According to still another embodiment the invention allows to predict the effect of drug or chemical or biological compound being put in presence of the cell and acting as a stimulus. Particularly the prediction concerns the main cellular funtions such as but not limited to cell cycle or cell division regulation, apoptosis, cell differentiation, DNA repair or synthesis, cell defence, activation of oncogene or antioncogene, tumor suppressor protein activity protease or proteinase, release, lipid metabolism, stress response, cell signalling, protein synthesis.

According to another embodiment, the cells, tissues or organisms are contacted with a substance of interest and the effect of the substance on the status/performance of the cell is monitored. The analysis performed according to the methods described here above allows a quantification of the changes within the cells compared to cells not contacted with the given compound. The invention is particularly useful to follow cellular reactions in the presence of biological or chemical compounds. Variations in the level of transcriptomes, proteomes, TF activation, Kinase activity, miRNA and/or DNA methylation are determined and give an overview of the changes occurring in the biological organisms, cells or tissues, in reaction to the presence of the compound.

The substance efficacy is preferably compared to the chemical structure of the compound in order to detect potential trends into the chemical parameters influencing the cell response to the substance. In another embodiment, compounds activity is evaluated for cell reaction after inhibition of a cell target. Thereafter, specific analysis based on data mining linking the various cellular, pathways and different levels of regulation provides the necessary information on the mechanism behind the presence of the given compound. Substance includes any chemical or biological molecules such as a drug or a molecule to investigate new pathways or as a potential drug. Substances also comprise biological molecules such as cytokines, growth hormones, or any biological molecules affecting cells. It also comprises chemical compounds such as drugs, toxic molecules and compounds from plants or animal extracts, chemicals resulting from organic synthesis including combinatory chemistry.

In one embodiment, cells, tissues or organisms are incubated in particular physical, chemical or biological conditions and the analysis is performed according to the present methods. The particular physical condition means conditions in which a physical parameter has been changed such as pH, temperature, pressure. The particular chemical conditions mean any conditions in which the concentration of one or several chemicals have been changed as compared to a control or reference condition including salts, oxygen, nutriments, proteins, glucides (carbohydrates), and lipids. The particular biological conditions mean any changes in the living cells, tissues or organisms including ageing, stress, transformation (cancer), pathology, which affect cells, tissues or organisms.

Therefore, the method as described herein may be utilized as part of a diagnostic and quantification kit which comprises means and media for performing an analysis of biological sample(s) containing target molecules being detected after their binding to the capture probes being present on the support in the form of arrays with a density of at least 5 different capture probes per cm² of surface of rigid support.

According to the invention, the solid support for the array is preferably selected from the group consisting of glass, metallic supports, polymeric supports (preferably a polystyrene support) or any other support used in the microchips (or micro-arrays) technology (preferably activated glass bearing aldehyde or epoxide or acrylate groups), said support comprising also specific coatings, markers or devices (bar codes, electronic devices, etc.) for improving the assay.

If glass presents many advantages (like being inert and having a low auto-fluorescence), other supports like polymers, with various chemically well-defined groups at their surface, allowing the binding of the nucleotide sequences are useful. One of the preferred support is the multi-well plates support including but not restricted to the 96, 384 or even 1536 well plates. In a preferred embodiment, the invention provides arrays in wells being part of multi-well plates having 24, 96 or 394 or even 1536 wells and containing the capture probes specific for performing the assays of the different cell components according to the present invention.

Miniaturization allows performing one assay onto a surface (usually circular spots of about 0.1 to about 1 mm diameter). A low density array, containing 20 to 400 spots is easily obtained with pins of 0.25 mm at low cost. Higher density of spots going to 1,600 spots per cm2 can be obtained by reducing the size of the spots for example to 0.15 mm. Method for obtaining capture molecules of higher density have been described earlier as in US 5,445,934. Miniaturization of the spot size allows obtaining a high number of data which can be obtained and analyzed simultaneously, the possibility to perform replicates and the small amount of biological sample necessary for the assay. Miniaturization for detection on microarrays is preferably associated with microfluidic substrate for separation, extraction of target molecules from the cell extract.

In a preferred embodiment, the assays for gene expression and TF are performed on arrays being on the same support or on different supports form cell extract coming from the same biological sample. Also in a preferred embodiment, the and/or kinase and possibly miRNA and/or cytokines and/or DNA methylation and/or SNP are performed on arrays being present on the same support or on different supports as the assays for gene expression and/or TF and on cell extract coming from the same biological sample.

In a preferred embodiment, the signal associated with a capture molecule on the array(s) is quantified. The preferred method is the scanning of the array(s) with a scanner being preferentially a laser confocal scanner such as "ScanArray" (Packard, USA) for the detection of fluorescent labeled targets. The preferred detection and/or quantification scanner for colorimetric analysis is performed with the silverquant scanner (Eppendorf, Hamburg, Germany). The resolution of the image is comprised between 1 and 500 µm and preferably between 5 and 50 µm. To Preferably the arrays is scanned at different photomultiplier tube (PMT) settings in order to maximize the dynamic range and the data processed for quantification and corrections with the appropriated controls and standards (de Longueville et al, Biochem Pharmacol. 64 , 2002,137-49).

The presence of target bound on the different capture probes present on the solid support may be analyzed, identified and quantified by an apparatus comprising a detection and quantification device of a signal formed at the location of the binding between the target molecule and the capture molecule, preferably also a reading device of information recorded on a surface of said solid support, a computer program for recognizing the discrete regions bearing the bound target molecules upon its corresponding capture molecules and their locations, preferably also a quantification program of the signal present at the locations and a program for correlating the presence of the signal at these locations with the diagnostic and the quantification of the components to be detected according to the invention.

According to an preferred embodiment the present invention provides a kit detecting and/or quantifying hierarchical (regulated) molecular change of a cell in response to an external biological, physical and/or chemical stimulus, which kit comprises at least at least 2 microarrays each one having at least 5 different capture probes being arranged at pre-determined locations of a solid support for the assay of the molecular change at first and/or second levels of the cell function, and optionally, buffers and labels.

In a preferred embodiment, the first microarray of the kit is dedicated to the detection and/or quantification of at least 10 different expressed genes present in the cell extract and the second microarray is dedicated to the detection and/or quantification of at least 5 different activated transcriptional factors and/or 5 different activated MAP-Kinases present in the cell extract.

In another embodiment, the first microarray of the kit is dedicated to the detection and/or quantification of at least 10 different expressed genes present in the cell extract and the second microarray is dedicated to the detection and/or quantification of at least 5 different cytokines present in the cell extract.

In an embodiment, the first microarray of the kit is dedicated to the detection and/or quantification of at least 10 different expressed genes present in the cell extract and the second microarray is dedicated to the detection and/or quantification of at least 5 different methylation sites of the DNA in the cell extract.

In one embodiment, the kit also include microarray comprising capture probes for a detection and a quantification of at least 5 different methylation sites and/or 5 SNP sites of the DNA in the same cell extract.

In a particular embodiment, the capture probes of the two microarrays are of different composition being polynucleotides and polypeptides. In another embodiment the capture probes are of the same composition (the capture probes have the same characteristics) for the detection of transcriptional factors and expressed genes and are polynucleotide sequences.

Preferably, the support provided in the kit contains a plurality of capture molecules capable to specifically bind at least 5 of the proteins as proposed in US 2004/0265938.

The kit also preferably contain reagent and means to perform the determination of cell transcriptional regulation in a sample, which kit comprises an array, harboring capture probes having a sequence identical or complementary to a miRNA or parts thereof and being present at pre-determined locations of the array, and buffers and labels.

The kit also preferably contains reagents and means to perform the evaluation of the activation level of cells by detection and/or quantification of the level of phosphorylation of multiple specific cellular proteins participating in signal transduction in response to a stimulus. The kit preferably comprises at least one support containing a plurality of immobilized capture molecules, said capture molecules being able to specifically bind to both the phosphorylated and non-phosphorylated forms of each cellular protein; two solutions for detection are provided, the first one being able of specifically binding to the phosphorylated target proteins but not to the non phosphorylated target proteins, the second one being able to specifically bind to the non-phosphorylated forms and possibly to the phosphorylated forms of target proteins; means for assessing the level of phosphorylation of said immobilized target proteins. According to a preferred embodiment either or both the capture molecules and the detection molecules are antibodies.

In another embodiment, the two microarrays of the kit are present on the same support or on different supports. In a preferred embodiment, the solid support is a 96 well plate and the different wells are used for performing the different assays of the molecular change at the two levels of cell function.

Preferably the polynucleotides and/or polypeptides are bound to the support at a concentration of at least about 0.01 pmole/cm.sup.2 of solid support surface; said specific nucleotide sequence are also preferably located at a distance of at least about 6.8 nm from the surface of the solid support.

Another aspect of the present invention is related to a diagnostic kit, including a kit of parts, possibly comprising said computer program or computer program product and comprising means and media for performing the steps b) and c) and possibly the step a) of the method according to the invention, said program is run on a computer. Therefore, all the results obtained from the detection and/or the quantification step, but also from various databases are collected and treated by a computer program which is preferably used for performing the steps e), f) and g) and possibly the step d)) of the method according to the invention.

Another aspect of the present invention is related to a computer program comprising a program codes means for performing the steps (steps e, f and g) and possibly the step d) of the method according to the invention, when said program is run on a computer.
The kit preferably comprise a storage media with the executable program being preferably in the form of hard drives, optical drives (CD-ROM, DVD-ROM, DVD-RAM), USB flash medium, DAT tape, ZIP drive, floppy disks of various formats 3.5 inches or 5.25 inches, an Ethernet virtual drive, directly mounted to the computer or remotely connected to said computer system.

In another embodiment, the kit contains a software for analysing the relationship between the identification and/or quantification of the miRNA (methylation, chromatin protection, SNP) present in the sample and the level of gene expressed in the same sample. In particular the software allows comparing the sequences of the miRNA with the sequence of the regulated genes.

The kit also preferably contains microarray support for making the assays of gene expression and transcriptional factor and/or kinase activity and possibly miRNA and/or cytokines and/or proteins. Preferably, the support is a glass slide. In another particular embodiment the support is a 24, 96 or 384-well plate with arrays being present in the wells. In another embodiment the multiwell plates which preferably contain 1536 wells contain a single capture probes per well. Preferably one multiwell plate is designed to perform at least 2 different types of assays.

Preferably, the invention is related also to a screening and/or quantification kit or device including high throughput screening device, possibly comprising computer-controllable electromagnetic means, microfluidic device or robots (such as high-throughput screening device) allowing the screening and detection upon any type of solid support and used for the screening and/or quantification of transcriptional factor(s) and of expressed genes present in a biological sample to said solid support bearing nucleotide sequences or polypeptides bound to the insoluble solid support.

### Examples

The following examples are provided for illustrative purpose only, and are not intended to limit the scope of the invention.

### 1. Hierarchical molecular change of a cell in response to IFNγ: analysis of activated transcription factors and gene expression levels on microarrays upon activation

### A. Cellular activation

U937 + interferonγ: cells are plated in 75 cm² flasks and grown in RPMI medium supplemented with 10% fetal bovine serum and antibiotics. They are stimulated at 10.10⁶ cells/75cm² flasks with interferonγ (1000U/ml) for 24 h or left untreated.

### B. Transcription factor (TF) microarrays

The TFChips MAPK kit was provided by Eppendorf (Hamburg, Germany). Transcription factor microarrays are present on glass slides. The arrays contain as capture probes double stranded DNA for the TF binding. Each transcription factor microarray is composed of triplicate spots. The TFChips MAPK allows the assay of AP1, STAT1, ELK1, P53, c-MYC, ATF2, NFAT and MEF2.

### C. Detection of activated transcription factor on TFChip MAPK.

A blocking step is performed in a Blocking Solution (TFChip MAPK kit, Eppendorf, Germany) for 1h at RT, followed by 1 washing with Washing Buffer A (TFChip MAPK kit, Eppendorf, Germany). The nuclear extracts are prepared as described in WO 01/73115. The nuclear extracts from the samples to be analyzed are diluted in a Sample Dilution Solution and Binding Solution (TFChip MAPK kit, Eppendorf, Germany) and contacted with the arrays. Incubation is performed for one h at Room Temperature and under agitation at 600 rpm. Samples are removed and microarrays are washed 3 times with Washing Buffer A (TFChip MAPK kit, Eppendorf, Germany).

A mix of antibodies (Primary Antibody Cocktail; TFChip MAPK kit, Eppendorf, Germany), each specifically recognizing the activated form of a transcription factor to be detected, is diluted in a Primary Antibody Solution (TFChip MAPK kit, Eppendorf, Germany), and contacted with the microarrays for 1h at RT, followed by washing 3 times with Washing Buffer A (TFChip MAPK kit, Eppendorf, Germany). Each microarray is then contacted with Cy3-labeled secondary antibodies under indirect light for 45 min at RT, followed by washing 3 times with Washing Buffer A (TFChip MAPK kit, Eppendorf, Germany).

Hybridization chambers are removed, and slides are rinsed with Washing Buffer B (TFChip MAPK kit, Eppendorf, Germany) and dried. A fluorescence detection of signals is performed by scanning slides using a laser confocal scanner "ScanArray" (Packard, USA) at a resolution of 10 µm. Signals are analyzed using the Eppendorf Image Analysis Software.

### D. Gene expression microarrays

The DualChip™ human RNAi (Eppendorf, Germany) side effect was used for assay of the changes in the gene expression.

The microarray contains 260 human genes in triplicates. In order to evaluate the entire experiment, several positive and negative controls (for hybridization and detection) as well as special capture probes for normalization. The DualChip™ consists of two microarrays on one slide that has already been framed with a hybridization frame.

The instructions provided by the supplier were followed for the hybridization and the analysis of the results.

### E. Analysis of gene expression

Total RNA was extracted from cells using the RNAgents kit (Promega) according to the manufacturer's protocol. 10 µg of total RNA sample was mixed with 2 µl oligo(dT)₁₂₋₁₈ (0.5 µg/µl, Roche), 3.5 µl H₂O, and 2 µl of a solution of 6 different synthetic well-defined poly(A+) RNAs. These latter served as internal standards to assist in quantification and estimation of experimental variation introduced during the subsequent steps of analysis. After an incubation of 10 min at 70°C and 5 minutes on ice, 9 µl of reaction mix were added. Reaction mix consisted in 4 µl Reverse Transcription Buffer 5X (Gibco BRL), 2 µl of DTT 0.1M, 1 µl RNAsin Ribonuclease Inhibitor (40 U/ml, Promega), and 2 µl of a 10X dNTP mix, made of dATP, dTTP, dGTP (5 mM each, Roche), dCTP (800 µM, Roche), and Biotin-11-dCTP (800 µM, NEN). After 5 min at room temperature, 1.5 µl SuperScript II (200 U/ml, Gibco BRL) was added and incubation was performed at 42°C for 90 minutes. Addition of SuperScript and incubation were repeated once. The mixture was then placed at 70°C for 15 minutes and 1 µl Ribonuclease H (2U/µl) was added for 20 minutes at 37°C. Finally, a 3 min denaturation step was performed at 95°C. The biotinylated cDNA, was kept at -20°C.

Hybridization mixture consisted in biotinylated cDNA (the total amount of labeled cDNA), 10 µl HybriBuffer A (Eppendorf, Hambourg, Germany), 40 µl HybriBuffer B (Eppendorf, Hambourg, Germany), 20 µl H₂O, and 10 µl of positive hybridization control.

Hybridization was carried out overnight at 60°C. The micro-arrays were then washed 4 times for 2 min with Unibuffer (Eppendorf, Hamburg, Germany).

The micro-arrays were than incubated for 45 min at room temperature with the Cy3-conjugated IgG Anti biotin (Jackson Immuno Research laboratories, Inc #200-162-096) diluted 1/1000 X Conjugate-Cy3 in the blocking reagent and protect from light.

The micro-arrays were washed again 4 times for 2 minutes with +Unibuffer and 2 times for 2 minutes with distilled water before being dried under a flux of N₂.

The hybridized micro-arrays were scanned using a laser confocal scanner "ScanArray" (Packard, USA) at a resolution of 10 µm. To maximize the dynamic range of the assay the same arrays were scanned at different photomultiplier tube (PMT) settings. After image acquisition, the scanned 16-bit images were imported to the software, 'ImaGene4.0' (BioDiscovery, Los Angeles, CA, USA), which was used to quantify the signal intensities. Data mining and determination of significantly expressed gene in the test compared to the reference arrays were performed according to the method described by Delongueville et al (Biochem Pharmacol. 2002 Jul 1; 64(1): 137-49). Briefly, the spots intensities were first corrected for the local background and than the ratios between the test and the reference arrays were calculated. To account variation in the different experimental steps, the data obtained from different hybridizations were normalized in two ways. First the values are corrected using a factor calculated from the intensity ratios of the internal standard reference and the test sample. The presence of 3 internal standard probes at different locations on the micro-array allows measurement of a local background and evaluation of the micro-array homogeneity, which is going to be considered in the normalization (Schuchhardt et al., Nucleic Acids Res. 28 (2000), E47). However, the internal standard control does not account for the quality of the mRNA samples, therefore a second step of normalization was performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained Ratios outside the 95% confidence interval were determined to be significantly changed by the treatment.

### F. Analysis of gene expression in relation with the TF activation

The data on the TF were related to the gene expression in the following way. The activated TF (in this model, STAT1) was incorporated into a data bank. The genes which were found activated were incorporated into another data bank. Then the data bank of the published paper of PubMed (http://www.ncbi.nlm.nih.gov/entrez/query.fcgi) was screened for the presence of genes already known to be associated with STAT1 and were isolated. After 24 h of activation, we found 13 genes already described as associated in the scientific papers or literature with the activation of STAT1 from the 83 genes found statistically significant (see table 1).

The promoters of the genes were screened for the presence of a sequence consensus for the binding of the activated
TF(http://molsun1.cbrc.aist.go.jp/research/db/TFSEARCH.html)
Out of 83 genes which were differentially expressed(see table 1), 52 contained the consensus binding sequence of STAT1.

The genes were classified according to their pathways with 15 genes in IFN response, 6 genes in apoptosis, 2 genes in cell adhesion, 9 genes in cell cycle, 2 genes in cell growth, 6 genes in DNA repair/synthesis, 4 genes in transcription, 3 genes in stress response, 9 genes in metabolism and still some other ones. The genes with the promoters containing the consensus sequence for the STAT1 binding were underlined and linked together.

Based on this association, STAT1 - the transcriptional factors detected to be activated -was linked to the 47 statistically significant genes containing the binding sequence of STAT1 to form a cluster of genes.

In a first level of analysis, the length of links between genes and regulated factor(s) (STAT1 in this example) are inversely proportional to the expression ratio. The greater the ratio is, the shorter the link displays the strength of the relation between the gene and STAT1. This diagram plots the hierarchical relationship between transcriptional factors and genes.

In a second level of analysis, a diagram relating the regulated factor(s) (STAT1 in this example) to a representation of the pathways containing the corresponding sequence is created. Pathway representation is a logical object organizing the pathway structure such as for example maps, matrix or database. Representation of the different pathways can be found for example on the Kyoto Encyclopaedia of Genes and Genomes (KEGG) website at http://www.genome.jp/kegg/. The length of the link is taken to be inversely proportional of the number of statistically significantly regulated genes in the pathway. A representation of each pathway is produced where the regulated genes (including the genes not containing the promoter sequence of the related transcriptional factor) are highlighted, for example with a color, depending on the strength (ratio) of the regulation. Thus, this diagram clearly expresses the influence of the transcriptional factor(s) on the different pathways through genes expression regulation.

This diagram expresses the effect of IFNγ on the transcription level and the gene level. It is possible to model the modification of the cells status triggered by IFNγ.

The diagram is completed by linking the transcriptional factor to all the genes of the presented pathways containing the related promoter sequence, even if the genes are not tested on the microarray. This permits to complete the model of the relationship between the transcriptional factor and the pathways.

### 2. Time frame for IFNγ stimulation on the activation of the cell.

The experiment was performed as described in example 1 with the cell being stimulated for 15 min or 24 h either with IFNγ at the same 1000u/ml.

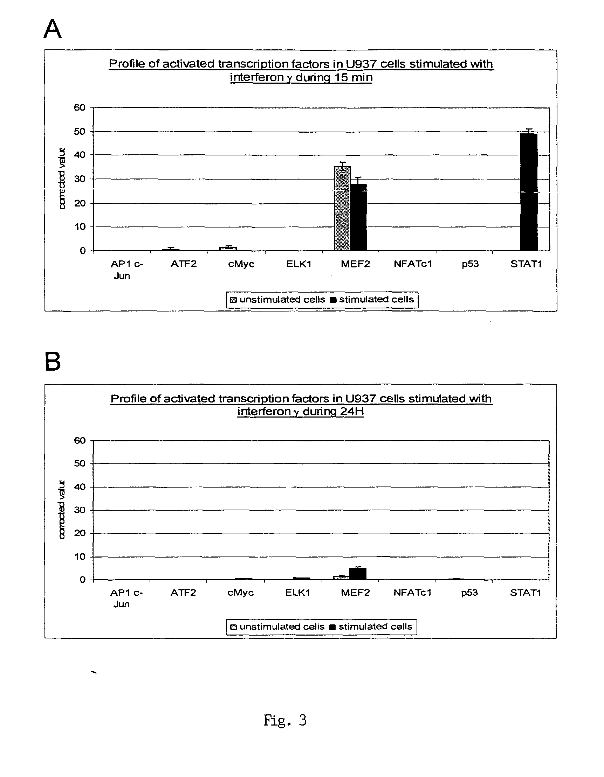

The assays were performed on the TFChips MAPK kit and on the DualChip™ human RNAi (Eppendorf, Germany) side effect. The results were analysed as proposed in example 1 and are presented in figure 3 and Table 1. In short the activation of the TF shows a fast activation of the STAT1 which disappear after 24h. MEF is activated in IFNγ stimulated and non stimulated conditions. MEF is sensitive to the handling and the state of the cells (confluency, low or high concentration of growth factor in culture medium.

The gene expression shows a large difference in the number and the genes which are over-expressed after 15 min and 24H. In brief, 11 genes on 260 were found over-expressed in a significant way after 15 min and 76 after 24H. From 11 over-expressed genes after 15 min, 5 genes were not any more over expressed after 24 h.

### Analysis of gene expression in relation with the TF activation

The activated TF (in this model, STAT1) were incorporated into a database. Then the data bank of the published paper of Medline (http://www.ncbi.nlm.nih.gov/entrez/query.fcgi) was screened for the presence of genes already known to be associated with each of the activated TF. We found 13 genes already described as associated with the activation of STAT1 from the 83 genes found statistically significant (see table 1).

The promoters of the genes were screened for the presence of a sequence consensus for the binding of the activated
TF(http://molsunl.cbrc.aist.go.jp/research/db/TFSEARCH.html.
52 genes from the 83 genes were found statistically significant, containing a sequence for the binding of STAT1 (see table 1).

Based upon the gene expression profile obtained at different time points, we are able to extract groups of genes having similar temporal behavior. Similarity between timecourse profile includes different meanings: genes having the same profile and/or genes having the same profile up to a normalization factor and/or genes having inverted profile and/or genes having the same profiles shifted in time. Depending on the definition of the similarity, a clustering method such as directed network models, K-means, hierarchical clustering, self-organizing maps, graph theory networks is used to create subgroups of genes.

At this stage, time course patterns related to group of genes are obtained. The next step is to assign them a biological meaning. This is done either by relating each group to a pathway or a group of pathways from a pathway database and/or by linking them to regulated transcriptional factor using the promoters database as explained in example 1.

### 3. Comparison of IFNα and IFNγ on the activation of the cell.

The experiment was performed as described in example 1 with the cell being stimulated for 24 H either with IFNγ or IFNα at the same 1000 /ml.

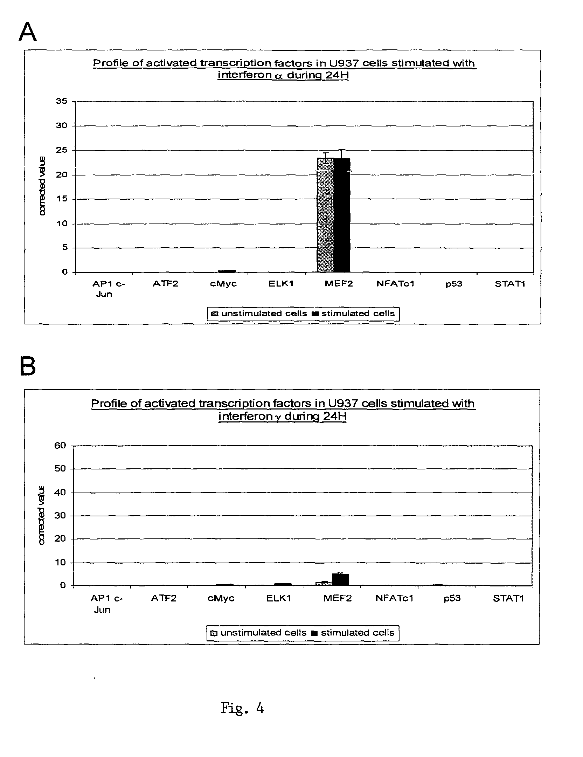

The assays were performed on the TFChips MAPK kit and on the DualChip™ human RNAi (Eppendorf, Germany) side effect. The results were analysed as proposed in example 1 and are presented in figure 4 and Table 2.
In short, the activation of the TF is similar in the two activation conditions. The same TF (STAT1) and many of the same genes are expressed (21 genes have the same gene expression profiling from 89 genes which have statistically significant gene expression values) with some being different.

### Analysis of gene expression in relation with the TF activation

The activated TF (in this model, STAT1) were incorporated into a data. Then the data bank of the published paper of Medline (http://www.ncbi.nlm.nih.gov/entrez/query.fcgi) was screened for the presence of genes already known to be associated with each of the activated TF were isolated. We found 12 genes already described as associated with the activation of STAT1 from the 89 genes found statistically significant (see table 1).

The promotors of the genes were screened for the presence of a sequence consensus for the binding of the activated
TF(http://molsun1.cbrc.aist.go.jp/research/db/TFSEARCH.html.
We found 52 genes from the 83 genes found statistically significant which contain a sequence for the binding of STAT1 (see table 1).

For both IFNα and IFNγ results, diagrams relating the activated transcriptional factors and gene expression profiles explained in example 1 can be created.

These diagrams depict the regulation of the transcriptional factor on the gene expression and relate that information to the involved pathways.

These diagrams allow to match the modification of the cells status triggered by IFNα et IFNγ. This is done by a correlation measure expressing the coincidence between the different models.

### 4. Hierarchical molecular change of a cell in response to β-estradiol and H₂O₂: analysis of activated transcription factors and gene expression levels on microarrays upon activation.

### A. cell activation

The cells used in this experiment are the MCF7. The activation with β-estradiol was performed in the following way: cells are plated in 75 cm² flasks and grown in RPMI medium supplemented with 10% fetal bovine serum and antibiotics. They are stimulated at 80% confluence with 1 µM β-estradiol for 4 h or 24h or left untreated as control.

For H₂O₂ treatment, the cells are plated in 75 cm² flasks and grown in RPMI medium supplemented with 10% fetal bovine serum and antibiotics. They are stimulated at 80% confluence with 200 µM H₂O₂ for 3 h or 24 h or left untreated as control.

### B. Detection of activated transcription factor on TFChip StemCell

In this assay, the TF were detected as in example 1 using TFChips MAPK kit but we also quantified the activity of the estradiol receptor alpha (ERα). The assays were performed on an array having spotted the double stranded DNA which is recognized by estradiol receptor. The capture probe contains a sequence specific for ERα.

The nuclear extracts from the samples are diluted in a binding buffer composed of HEPES buffer supplemented with salt, EDTA, MgCl₂, glycerol, protease and phosphatase inhibitors, and contacted with the arrays. Incubation is performed for 1 h at room temperature and under agitation at 600 rpm. Samples are removed and microarrays are washed. The assay was performed as described in example 1 using antibodies specific for ERα (Santa Cruz,sc-8005). The antibodies, specifically recognizing the activated form of ERα is diluted in a Primary Antibody Solution (Eppendorf, Germany), and contacted with the microarrays for 1h at room temperature, followed by washing 3 times with Washing. Each microarray is then contacted with cy3-labeled secondary antibodies under indirect light for 45 min at room temperature, followed by washing. Hybridization chambers are removed, and slides are rinsed with a phosphate buffer and dried. A fluorescence detection of signals is performed by scanning slides using a laser confocal scanner "ScanArray" (Packard, USA) at a resolution of 10 µm. Signals are analyzed using the Eppendorf Image Analysis Software.

In short, the activation of the TF with β-estradiol shows a fast activation of the Era which disappears after 24h. This activation shows a greater activation for c-MYC after 4 h than after 24h. p53 is activated in stimulated and non stimulated conditions. p53 is sensitive to the confluency of the cells. The β-estradiol stimulates the cellular proliferation and after 24 h with β-estradiol, the cells are very confluent and the activation of p53 is high. The stimulation with H₂O₂ shows an activation of p53 after 3 h or 24 h of stimulation.

### C. Detection of gene expression

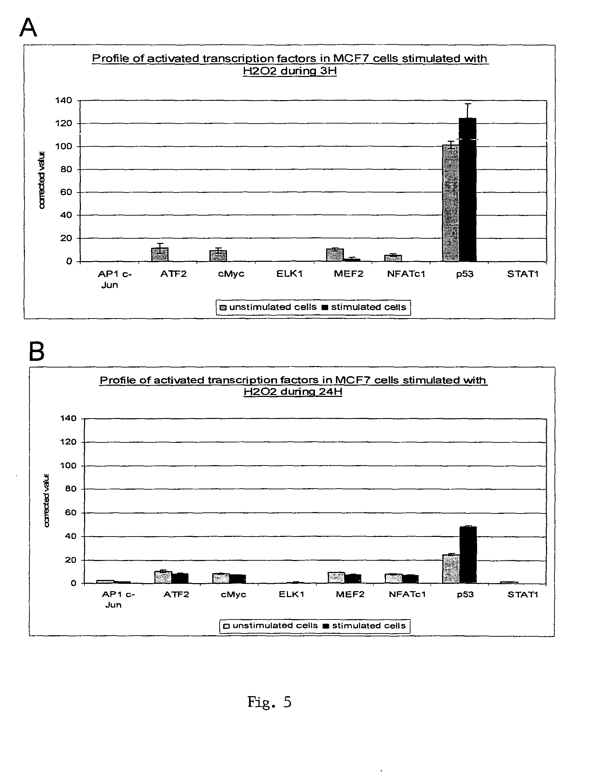

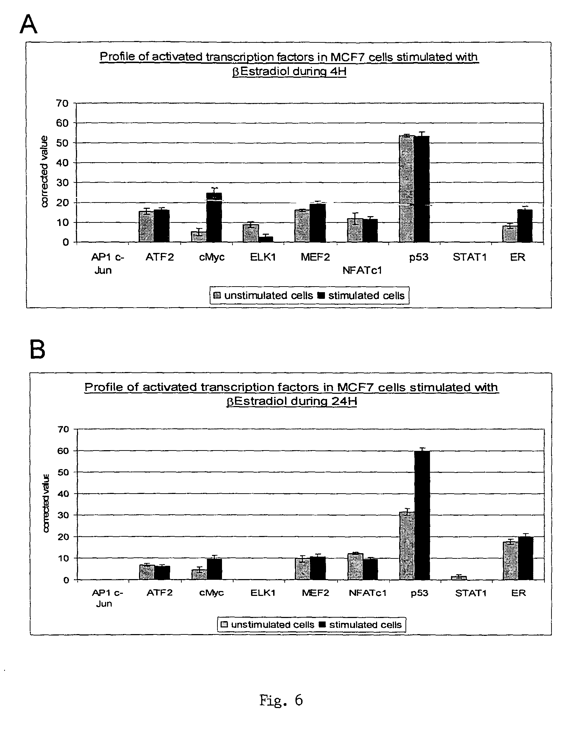

In this experiment, The DualChip™ human breast cancer was used to determine gene expression changes in MCF7 cells incubated in the presence of 1 µM β-estradiol during 4h and 24 hours or with H₂O₂ during 3 h and 24 h in comparison to control cells. The DualChip™ human breast cancer (Eppendorf, Germany) was used and The microarray contains 210 human genes in triplicates. Hybridizations were carryout out in triplicate and the quantification performed as explained in example 1. The results are presented in figure 5, figure 6 and Table 3.

### D. Analysis of gene expression in relation with the transcriptional factor activation.

The analysis was performed as proposed in example 3 with matches of the modification of the cell status triggered by β-estradiol and H₂O₂.

## Claims

1. A method for detecting and/or quantifying hierarchical molecular change of a cell in response to an external biological, physical and/or chemical stimulus, by integrating at least a first level of a cell function and a second level of a cell function, the molecules involved in the first level being regulatory molecules interacting with the expression of the molecules of the second level, by the following steps:
a) obtaining a cell extract from the cell;
b) determining and/or quantifying the molecular change at a first level of the cell function by submitting the cell extract to a first assay upon microarray, said first assay capable of obtaining :
- a detection and/or a quantification of at least 5 different (activated) transcriptional factors present in the cell extract and/or,
- a detection and/or a quantification of the activity of at least 5 different MAP-kinases;
c) detecting and/or quantifying the molecular cell changes at a second level of a cell function, by submitting the same cell extract to a second assay upon microarray, said second assay comprising:
- a detection and/or a quantification of at least 10 different expressed genes and/or,
- a detection and/or a quantification of at least 10 different corresponding proteins encoded by these expressed genes, wherein the expressed genes and the corresponding synthesized proteins are regulated by the (activated) transcriptional factors and MAP-kinases of the first level of the cell function,
d) collecting the results of detection and/or quantification of the expressed genes and/or synthesized proteins to the results of the detection and/or the quantification of the (activated) transcriptional factors and/or activated MAP-kinases;
e) correlating the said results of the detection and/or the quantification of the expressed genes and/or synthesized proteins to the said results of the detection and/or the quantification of the (antivated) transcriptional factors and/or activated MAP-kinases;
f) integrating the results of said detection and/or quantification and their correlation in a model of a hierarchical system, preferably by a computer and possibly,
g) characterizing, preferably by a computer, the hierarchical molecular change in the cell resulting from the external biological, physical and/or chemical stimulus.

2. The method according to claim 1, wherein the detection and/or quantification of hierarchical molecular change of a cell in response to an external biological, physical and/or chemical stimulus is obtained by comparing the detection and/or quantification of the cell response to a detection and/or quantification of a control cell which has not been submitted to the stimulus.

3. The method according to claim 1 or 2, wherein the external biological stimulus is a binding of one or more ligands to receptor(s) of the cell.

4. The method according to any of the claims 1 to 3, wherein the expressed genes detected and/or quantified are selected among a multiplicity of at least 100, preferably at least 200, more preferably at least 1000 genes, encoded in the genome of the cell and wherein said detection and/or quantification is obtained by the steps of:
a) obtaining a cell extract containing a pool of target nucleic acids comprising RNA transcripts of one or more of the said genes, or nucleic acids derived therefrom using said RNA transcripts as templates;
b) hybridizing said pool of target nucleic acids to a microarray of polynucleotide capture probes immobilized on a solid support surface, wherein each different polynucleotide is localized at a predetermined location of the said solid support surface and,
c) detecting and/or quantifying the hybridization of the said nucleic acids to said polynucleotide probes of the microarray and wherein the quantification is proportional to the expression level of the said genes.

5. The method according to claim 4, wherein the quantitative determination of the expression of the multiplicity of genes is performed on genes belonging to or being representative of at least 9 of the vital cellular functions selected from the group consisting of: apoptosis, cell adhesion, cell cycle, growth factors and cytokines, cell signalling, chromosomal processing, DNA repair/synthesis, intermediate metabolism, extracellular matrix, cell structure, protein metabolism, oxidative metabolism, transcription, and house keeping genes, said functions being represented by at least 4 different genes.

6. The method according to any of the claims 1 to 5, wherein the detection and/or the quantification of the different (activated) transcriptional factors is performed by the steps of:
a) obtaining a cell extract containing activated transcriptional factors;
b) contacting the cell extract under conditions allowing the binding of (activated) transcriptional factors with a microarray made of at least 5 capture probes/cm² of the solid support surface, wherein each different capture probe is localized in a predetermined location of the said solid support surface, wherein each capture probe is specific of the corresponding transcriptional factors, and
c) detecting and/or quantifying signal(s) resulting from the binding of the (activated) transcriptional factors to their corresponding capture probes, and wherein the location of the signal is related to the transcriptional factor identity (detection and/or quantification) present in the said location.

7. The method according to claim 6, wherein the capture probes are double-stranded DNA sequences immobilized on the solid support surface at a concentration of at least 0.01 micromoles/cm², and wherein the double-stranded DNA sequences comprise a specific sequence able to bind specifically an (activated) transcriptional factor and wherein the double-stranded DNA sequences are linked to the solid support surface by a spacer having a length of at least 6.8 nm.

8. The method according to any of the claims 1 to 7, wherein the quantitative of the activity of the different activated MAP-kinases, is performed by the steps of :
a) obtaining a cell extract containing activated MAP-kinases and phosphatases;
b) determining the equilibrium between the activities of kinase/phosphatase enzymes on proteins participating in signal transduction into cells or by quantitatively determining the level of phosphorylation of cellular proteins participating in signal transduction belonging to a cascade of phosphorylation leading to the activation of at least one transcriptional factor.

9. The method according to any of the claims 1 to 8, wherein the first assay further comprises a detection and/or a quantification of at least 5 different miRNA.

10. The method according to claim 9, wherein the detection and/or quantification of the different miRNA is performed by the steps of:
a) obtaining a cell extract containing a pool of miRNA;
b) elongating or copying or ligating said miRNAs into target labeled polynucleotides;
c) hybridizing said target labeled polynucleotides to a microarray of polynucleotide capture probes immobilized on a solid support surface, wherein each different polynucleotide is localized at a predetermined location of the said solid support surface, and wherein each capture probe is specific of a corresponding labelled polynucleotide;
d) detecting and/or quantifying signals resulting from the binding of the target labeled polynucleotides to the polynucleotide probes wherein the location of the signal is related to the miRNA identity (detection and/or quantification) present in the said location.

11. The method according to any of the claims 1 to 10, wherein the first assay further comprises a detection and/or a quantification of at least 5 different cytokines.

12. The method according to claim 11, wherein the detection and/or quantification of the cytokines is performed by the steps of:
a) obtaining a cell extract containing a pool of cytokines;
b) contacting the cell extract under conditions allowing the binding of cytokines with a microarray comprising at least 5 different capture probes being antibodies, immobilized on a solid support surface, wherein each different antibody is localised at a predetermined location of the said solid support surface and wherein each antibody is specific of the corresponding cytokine;
c) detecting and/or quantifying signals resulting from the binding of the cytokines to the capture probes wherein the location of the signal is related to the cytokine identity (detection and/or quantification) present in the said location.

13. The method according to any of the claims 1 to 12, wherein the first assay further comprises a detection (and/or a quantification) of at least 5 and preferably 20 different methylation sites of a DNA sequence, a detection and/or quantification of a chromatin protection assay, and/or a detection (and/or a quantification) of at least 5 and preferably at least 20 different single nucleotide polymorphisms sites of a DNA sequence.

14. The method according to any of the claims 1 to 13, wherein the at least 10 different proteins encoded by the expressed genes are assayed on an microarray of captured probes immobilized on a solid support surface and wherein the detection and/or quantification of the proteins is determined by a signal resulting from one characteristic specific of the proteins and wherein the signal is quantified.

15. The method according to any of the claims 1 to 14, wherein the at least 10 different proteins encoded by the expressed genes are assayed after separation in a 2D gel electrophoresis.

16. The method according to claims 14 and 15, wherein the signal resulting from one characteristic specific of the proteins is the binding of an antibody against an epitope of the protein.

17. The method according to claims 14 and 15, wherein the at least 10 different proteins are detected by their molecular weight, preferably determined by a mass spectrometer, more preferably by a MALDI spectrometer.

18. The method according to claim 17, wherein the protein is digested into peptides before their detection and/or quantification by the mass spectrometer analysis.

19. The method according to any of the claims 1 to 18, which further comprises a receptor activation assay.

20. The method according to any of the claims 1 to 19, which further comprises a receptor activation assay of a receptor that activates kinases selected from the group consisting of serine, threonine or tyrosine kinase enzymes.

21. The method according to any of the claims 1 to 20, wherein the capture probes present on the array have a density of higher than 20, preferably higher than 400 and even higher than 1,600 spots per cm² of the solid support surface.

22. The method according to any of the claims 1 to 21, which further comprises a modelling step of identification of key regulatory element responsible for changes occurring under a particular stimulus.

23. The method according to any of the claims 1 to 22, wherein the determination and/or quantification of molecular change at first and second levels of cell function are determined by assays performed on microarrays bearing capture probes being of different or the same composition selected from the group consisting of polynucleotides and polypeptides.

24. The method according to any of the claims 1 to 23, wherein the determination and/or quantification of molecular change at first and second levels of the cell function are determined by assays performed in wells being part of multi-well plates having preferably a 24, 96 or 384 or even 1536 wells format.

25. The method according to any of the claims 1 to 24, wherein the determination and/or quantification of molecular change at first and second levels of cell function are determined by assays performed on beads.

26. The method according to any of the claims 1 to 25, wherein the molecular change of a cell in response to an external biological, physical or chemical stimulus are analyzed over a time period.

27. The method according to any of the claims 1 to 26, wherein the step e) of correlating the results is obtained by an independent analysis of the two level data and a representation of interactions between said levels, wherein said comparison highlights the regulation of the biological process on said regulated process.

28. The method according to any of the claims 1 to 27, wherein the step e) of correlating the results is obtained by a comparison of the results with a database, which comprises information related to molecules involved in the first level, as regulatory molecules interacting with the expression of molecules of the second level, preferably pathway network annotations present in the patent and scientific papers, describing these molecules.

29. The method according to claim 28, wherein the data are the results of detection and/or quantification of the first level and second level molecules (relative) abundance values or profiles.

30. The method according to claim 29, wherein the data are the results of detection and/or quantification obtained from an analysis of the cell extracts upon 4 arrays, comprising at least one array for the detection and/or the quantification of different expressed genes and/or proteins and at least one array for the detection and/or the quantification of different activated transcriptional factor and/or different activated MAP-kinases, and at least one array for the detection and/or the quantification of molecules selected from the group consisting of miRNA and/or different methylation sites and/or SNP sites and/or cytokines.

31. The method according to claim 29, wherein the data are the results of detection and/or quantification obtained from an analysis of the cell extracts upon 5 arrays, comprising at least one array for the detection and/or the quantification of different expressed genes and/or proteins and at least one array for the detection and/or the quantification of different activated transcriptional factor and/or different activated MAP-kinases, and at least one array, preferably at least two arrays, for the detection and/or the quantification of molecules selected from the group consisting of miRNA and/or different methylation sites and/or SNP sites and/or cytokines.

32. The method according to any of the claims 1 to 31, wherein the steps e) and f) of integrating the results of the detection, quantification and correlation in a model is obtained by creating a relation network made of nodes representing molecules of the first level and molecules of the second level, and directed and undirected links between these nodes present in patents and scientific papers, describing the interactions between these molecules.

33. The method according to claim 32, wherein the model is a model of interactions between the two levels of cell function constructed by processing the results at once, by computing scores of correlation between the first level and the second level.

34. The method according to claim 32, wherein a delay parameter is introduced between the detection and quantification steps to account for a response delay between levels (layers) of a response.

35. The method according to claim 32, wherein the relation network is complemented by data obtained from an additional database comprising data selected from the group consisting of validated interaction models between the two levels, previous computed pathway mapping on known regulatory biological processes and computed interaction models from previous experiments.

36. The method according to claim 32, wherein the relation network is made of random network, scaled-free network or hierarchical network describing on the organisation of the said nodes and links.

37. The method according to claim 32, wherein the executable program constructs a hierarchical model of the effect of the regulatory biological process on the second level by using models and networks selected from the group consisting of directed network models, K-means, hierarchical clustering, self-organizing maps, neural networks, Bayesian networks, Gaussian graphical models, co-expression networks using conditional independence, graph theory networks.

38. The method according to claim 29, wherein the data are the results of detection and/or the quantification obtained from an analysis of the cell extracts upon 5 arrays comprising at least one array on different expressed genes and/or protein and at least one array on different activated transcriptional factor and/or different activated MAP-kinases and at least two arrays on miRNA and/or different methylation sites and/or SNP sites and/or cytokines.

39. The method according to claim 32, wherein the relational network of nodes arc clustered in subsets of nodes said nodes representing entities from said levels and directed or undirected links between said nodes evaluating biological interactions and are connected in specific diagrams and represented as squares, triangles, and/or polygons.

40. Use of the method according to any of the claims 1 to 39 for the detection of a potential molecule target in the cell for drug development.

41. Use of the method according to any of the claims 1 to 39 for protecting the effect of inhibition or the activation of one or more elements selected from the group consisting of a kinase, a phosphatase, a transcriptional factor, a receptor on the hierarchical molecular change of a cell in response to an external, biological, physical and/or chemical stimulus.

42. Use of the method according to any of the claims 1 to 39 for modelling the effect of inhibition or activation of molecules selected from the group consisting of a kinase, a phosphatase, a transcriptional factor or a receptor on the hierarchical molecular change of the cell in response to an external, biological, physical and/or chemical stimulus.

43. Use of the method according to any of the claims 1 to 39, for predicting the effect of a drug or a chemical or a biological compound being put in presence of the cell and acting as a stimulus.

44. Use of the method according to any of the claims 1 to 39 in synthetic biology.

45. A computer program comprising the program code means for performing the steps e) f), g) (and possibly d)) of the method according to any of the claims 1 to 39, wherein said program is run on a computer.

46. A computer program product comprising the program code means stored on a computer readable medium for performing the steps e), f), g) (and possibly d)) of the method according to any of the claims 1 to 39, when said program is run on a computer.

47. A kit or device for detecting and/or quantifying hierarchical molecular change of a cell, in response to an external biological, physical and/or chemical stimulus, comprising the computer program or computer program product of claim 45 or 46 and at least 2 microarrays for performing the steps b) and c) and possibly a) of the method according to any of the claims 1 to 39, wherein each microarray comprises at least 5 different capture probes being arranged at pre-determined locations of the microarray solid support surface for the assay of molecular change of a first and second level of the cell function, and optionally, buffers and labels.

48. The kit or device according to claim 47, wherein the first microarray comprises capture probes for a detection and/or a quantification of at least 10 different expressed genes present in an extract of the cell and wherein the second microarray comprises capture probes for a detection and/or a quantification of at least 5 different activated transcriptional factors and/or 5 different activated MAP-kinases present in the same cell extract.

49. The kit or device according to claim 47 or 48, wherein the first microarray comprises capture probes for a detection and/or a quantification of at least 10 different expressed genes present in an extract of a cell, and wherein the second microarray comprises capture probes for:
- a detection and/or a quantification of at least 5 different miRNA present in the same cell extract, or for a detection and/or a quantification of at least 5 different cytokines present in the same cell extract,
- a detection and/or a quantification of at least 5 different methylation sites of a DNA sequence or a detection and/or quantification of a chromatin protection assay, or a detection and/or quantification of at least 5 different single nucleotide polymorphisms sites of a DNA sequence present in the same cell extract.

50. The kit or device according to any of the claims 47 to 49, wherein the two microarrays are present on the same solid support.

51. The kit or device according to any of the claims 47 to 49, wherein the two microarrays are present on different solid supports.

52. The kit or device according to any of the claims 47 to 51, wherein the capture probes of the two microarrays are of different composition.

53. The kit or device according to any of the claims 47 to 51, wherein the capture probes of the two microarrays are of the same composition.

54. The kit or device according to claim 53, wherein the capture probes are polynucleotide sequences.

55. The kit or device according to any of the claims 47 to 54, wherein the solid support is a multiple well plate, preferably a 24, a 96, a 384 or a 1536 well plate.

56. The device according to any of the claims 47 to 55 which is a high throughput screening device.
